(19) **Europäisches Patentamt / European Patent Office / Office européen des brevets**

(11) **EP 3 997 242 B1**

(12) **EUROPEAN PATENT SPECIFICATION**

(45) Date of publication and mention
of the grant of the patent:
**08.04.2026   Bulletin 2026/15**

(21) Application number: **20836094.1**

(22) Date of filing: **09.07.2020**

(51) International Patent Classification (IPC):
*C12Q 1/6809* (2018.01)   *C12Q 1/6869* (2018.01)
*G16B 40/00* (2019.01)

(52) Cooperative Patent Classification (CPC):
**C12Q 1/6883; C12Q 1/6809; G16B 20/00**

(86) International application number:
**PCT/US2020/041469**

(87) International publication number:
**WO 2021/007462 (14.01.2021 Gazette 2021/02)**

(54) **METHODS OF DETECTING DISEASE AND TREATMENT RESPONSE IN CFDNA**

VERFAHREN ZUR DETEKTION VON KRANKHEITEN UND ZUR BEHANDLUNGSREAKTIONEN IN CFDNA

MÉTHODES DE DÉTECTION DE MALADIE ET DE RÉPONSE AU TRAITEMENT DE CFADN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2019   US 201962872234 P
19.08.2019   US 201962888997 P**

(43) Date of publication of application:
**18.05.2022   Bulletin 2022/20**

(73) Proprietor: **The Translational Genomics Research
Institute
Phoenix, Arizona 85004 (US)**

(72) Inventors:
• **MURTAZA, Muhammed**
  Phoenix, Arizona 85004 (US)
• **MCDONALD, Bradon**
  Phoenix, Arizona 85004 (US)
• **MARKUS, Havell**
  Phoenix, Arizona 85004 (US)

(74) Representative: **Barker Brettell LLP
100 Hagley Road
Edgbaston
Birmingham B16 8QQ (GB)**

(56) References cited:
**WO-A1-2018/009723     US-A1- 2017 342 475**

• CHRISTOPHER K. RAYMOND ET AL: "Collection
of cell-free DNA for genomic analysis of solid
tumors in a clinical laboratory setting", PLOS
ONE, vol. 12, no. 4, 27 April 2017 (2017-04-27),
pages 1 - 11, XP055595111, DOI: 10.1371/
journal.pone.0176241
• XIANGYUAN MA ET AL: "Cell-Free DNA Provides
a Good Representation of the Tumor Genome
Despite Its Biased Fragmentation Patterns",
PLOS ONE, vol. 12, no. 1, 3 January 2017
(2017-01-03), pages e0169231, XP055523795,
DOI: 10.1371/journal.pone.0169231
• HARVELL MARKUS ET AL: "Analysis of
recurrently protected genomic regions in cell-
free DNA found in urine", SCI. TRANSL. MED, 17
February 2021 (2021-02-17), XP055898119,
Retrieved from the Internet <URL:https://www.
science.org/doi/epdf/10.1126/scitranslmed.
aaz3088> [retrieved on 20220307]
• BUDHRAJA KARAN K. ET AL: "Genome-wide
analysis of aberrant position and sequence of
plasma DNA fragment ends in patients with
cancer", SCIENCE TRANSLATIONAL MEDICINE,
vol. 15, no. 678, 11 January 2023 (2023-01-11),
XP093056866, ISSN: 1946-6234, DOI: 10.1126/
scitranslmed.abm6863

## Description

### CROSS-REFERENCE TO RELATED APPLICATIONS

This application claims priority to U.S. Provisional Application No. 62/872,234, filed July 9, 2019, and U.S. Provisional Application No. 62/888,997, filed August 19, 2019.

### TECHNICAL FIELD

The present invention relates to a method for detecting an aberrant end cell free DNA (cfDNA) fragmentation profile of a subject .

### BACKGROUND

Circulating cell-free DNA (cfDNA) has emerged as an informative biomarker in prenatal, organ transplant and cancer patients. Recent studies have shown that genome-wide distribution and fragmentation of cfDNA in plasma is not random. Plasma cfDNA fragments have a modal size of 167 base pairs (bp), are protected from degradation within mono-nucleosomes and their positioning captures nucleosome footprints of contributing tissues[1]. In cancer patients, these observations potentially enable cancer detection[2], inference of tissue of origin[3] and inference of gene expression[4]. In addition, deviations from expected fragment size and positioning can be leveraged to improve signal-to-noise ratio for somatic genomic alterations in plasma cfDNA[5].

The mainstay of cancer management is drug therapy, including cytotoxic chemotherapy, molecularly targeted treatment, and endocrine therapy. Such treatments are not curative and the clinical goal is to prolong life, alleviate symptoms, and preserve quality of life.

Thus, a need exists for methods of early detection or recurrence of disease in a subject and the continual monitoring of treatment and/or disease progression. More specifically, a need exists for early detection methods of diseases, such as, cancer, preferably using non-invasively collected biological samples, such as, urine. Collection of blood plasma requires venipuncture and plasma volume obtainable at a single time point is limited. In contrast, if a reliable method could be developed based on a urine sample, it would have the advantage of being noninvasively collected, with minimal assistance, and in larger volumes. To date, compared to plasma, there is no reliable urine cfDNA analysis. Problems with using urine samples to date include cfDNA fragments being degraded, shorter and of variably size[6,7]. These problems have impeded the use of urine for targeted analysis of genomic alterations. Comprehensive characterization of fragment sizes and positioning in urine cfDNA has not been reported and whether any genome-wide organization is preserved is unknown to date. Raymond et al., 2017, PLOS ONE, (DOI: 10.1371/journal.poe.0176241) discloses a method of determining an aberrant end cfDNA fragmentation profile of cfDNA isolated from samples of individuals by next generation sequencing.

### SUMMARY

The invention is as defined in the appended claims. The present disclosure provides several tools for increasing the sensitivity and analytical precision of the disclosed methods for monitoring cfDNA. cfDNA relates to nucleic acids released by cells into the circulation that have a half-life of roughly 1 to 2 hours. The present dislcosure employs whole genome sequencing (WGS) of cfDNA in a sample to detect disease and/or to infer contributing cell types in urine cfDNA. The present disclosure also provides for real-time treatment response monitoring in patients with a disease. The present disclosure additionally leverages the potential of urine cfDNA as a non-invasive, real-time indicator of disease burden and patient response to treatment.

A method of generating/detecting a cfDNA profile of a subject is disclosed herein. In certain embodiments the method comprises: a) obtaining a sample from the subject; b) extracting cfDNA from the sample to obtain cfDNA fragments; c) performing WGS on the cfDNA fragments extracted from the sample to generate sequencing reads for the cfDNA fragments; d) determining from the sequencing reads a distribution of start and end sites of the cfDNA fragments to generate/dectect the subject's cfDNA fragmentation profile.

In another aspect, the disclosure is directed to a method of detecting disease in a subject by comparing a subject's cfDNA profile to a control sample. The method comprises the steps of: In other aspects, the disclosure provides a method of detection of disease in a subject through sequencing and analysis of cfDNA, the method comprising: a) obtaining a sample from the subject; b) extracting cfDNA from the sample to obtain cfDNA fragments; c) performing WGS on the cfDNA fragments extracted from the sample to generate sequencing reads for the cfDNA fragments; d) determining from the sequencing reads a distribution of start and end sites of the cfDNA fragments to generate the subject's fragmentation profile; e) comparing the subject's cfDNA fragmentation profile to a fragmentation profile of cfDNA from a control sample; and f) detecting the presence of disease in the subject based on the subject's fragmentation profile deviating from the control sample cfDNA fragmentation profile.

In yet other aspects, the disclosure is directed to a method of detecting a cancer subtype in a subject. The method comprises the steps of: a) obtaining a sample from the subject; b) extracting cfDNA from the sample to obtain cfDNA fragments; c) performing whole genome sequencing on the cfDNA fragments extracted from the sample to generate sequencing reads for the cfDNA fragments; d) determining from the sequencing reads a distribution of start and end sites of the cfDNA fragments to generate the subject's fragmentation profile; e) comparing the subject's cfDNA fragmentation profile to the cfDNA fragmentation profile from a control sample; and f) detecting presence of a cancer subtype in the subject based on the subject's cfDNA fragmentation profile deviating from the control sample cfDNA fragmentation profile.

In other aspects, the disclosure is directed to a method of monitoring a subject's response to treatment for a disease. The method comprises the steps of: a) obtaining a first sample from the subject prior to administering a treatment to the subject; b) obtaining a second sample from the subject after administering the treatment to the subject; c) extracting cfDNA from the first sample to obtain cfDNA fragments; d) extracting cfDNA from the second sample to obtain cfDNA fragments; e) performing whole genome sequencing on the first and second sets of cfDNA fragments extracted from the samples to generate sequencing reads for the first and second set of cfDNA fragments; f) determining from the sequencing reads a distribution of start and end sites of the cfDNA fragments to generate the subject's first and second fragmentation profile; g) comparing the subject's first and second fragmentation profiles to a fragmentation profile of a reference sample; and h) determining the subject's response to the treatment based on the similarity or difference in the subject's first and second cfDNA fragmentation profile compared to a reference sample cfDNA fragmentation profile.

In a particularly advantageous embodiment, the samples from the subject and the control sample are urine samples.

In specific non-limiting embodiments, the generation of the cfDNA fragmentation profile further comprises determining a nucleotide frequency at the start and end sites of the cfDNA fragments, the determination of the aberrant ends fraction in the cfDNA fragments, the generation of a nucleosome map and determination of nucleosome peaks, the median fragment length, and the coverage at transcriptional start sites.

## BRIEF DESCRIPTION OF THE DRAWINGS

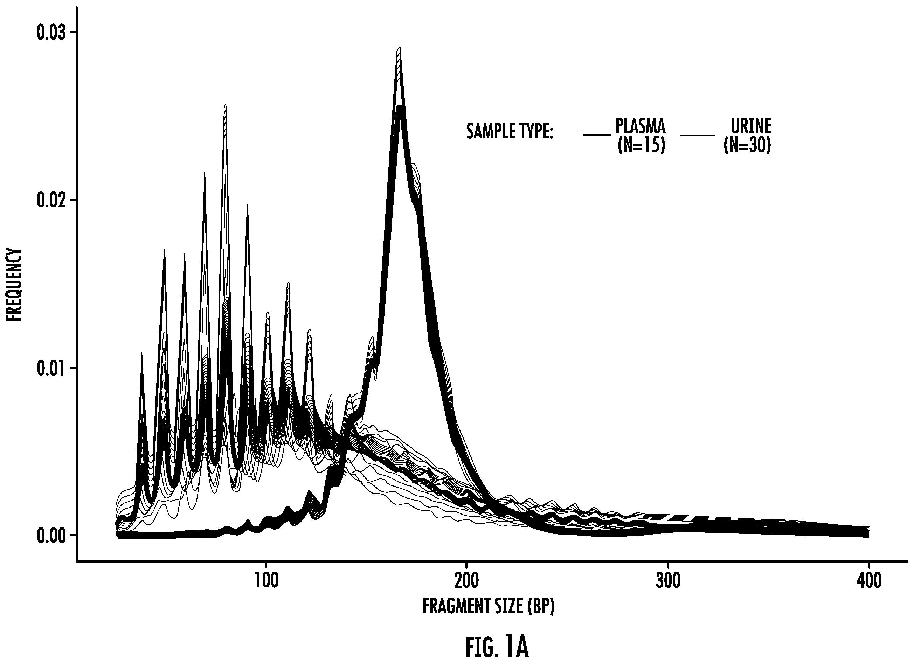

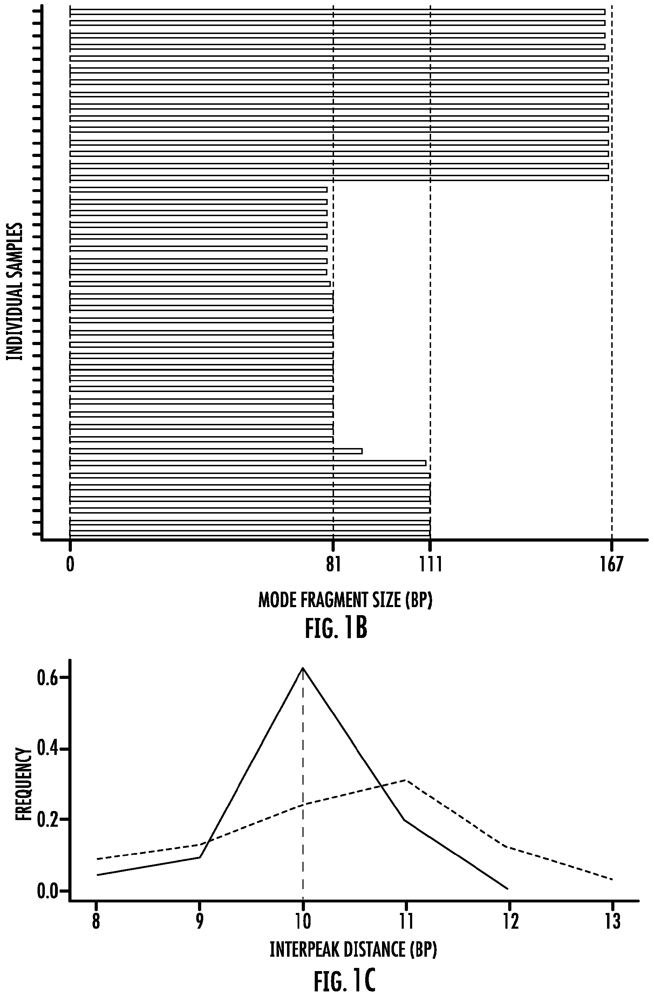

FIGs 1A-1C depict the comparison of fragment size between plasma and urine samples. In **FIG. 1A** genome-wide fragment size distributions were measured. Individual control plasma and urine samples are shown as grey lines. Mean across plasma and urine samples are shown as red and yellow lines, respectively. Modal size in individual control plasma and urine samples was defined as the fragment size with the highest frequency in **FIG. 1B. FIG. 1C** illustrates interpeak (peak-to-peak) distance of periodic peaks for plasma and urine samples.

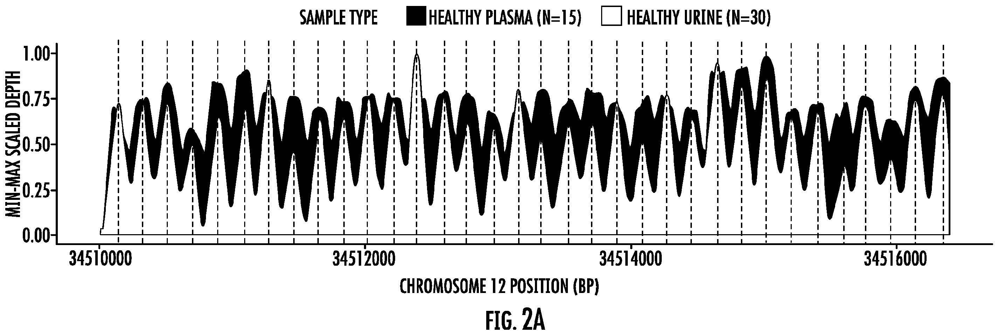

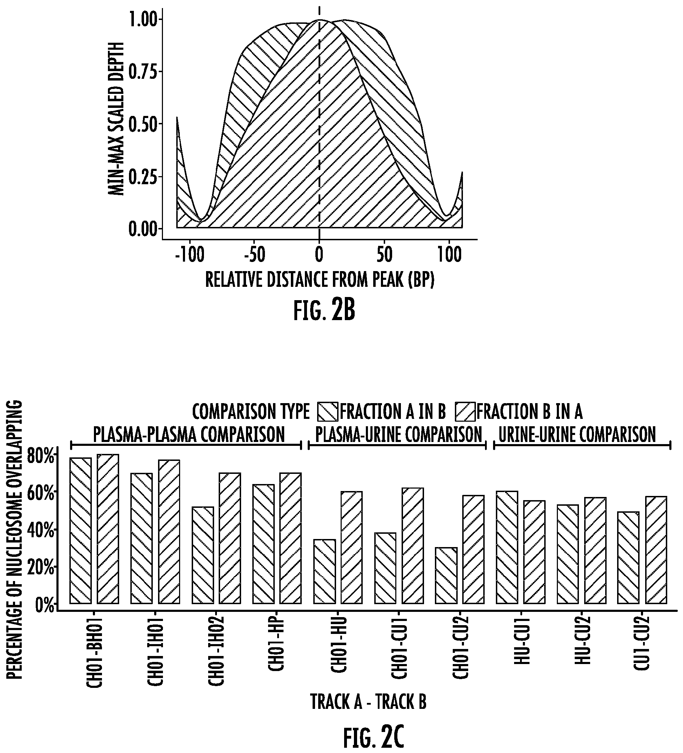

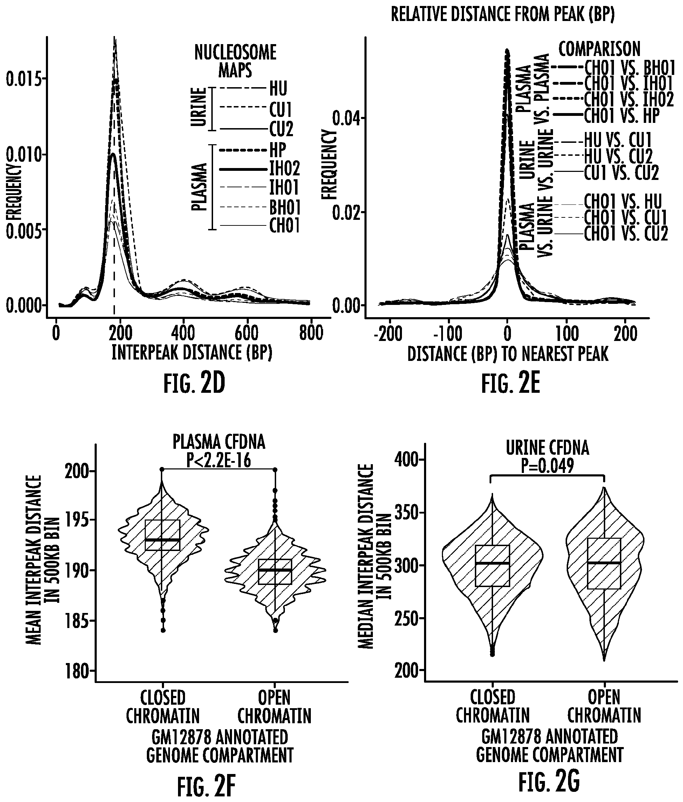

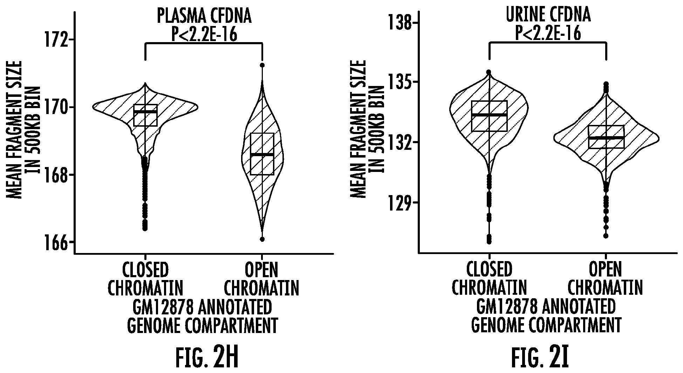

FIGs 2A-2I show the relationship between nucleosome positioning and sequencing coverage of cfDNA fragments in plasma and urine samples. **FIG. 2A** depicts the LOESS smoothed and min-max scaled physical sequencing coverage of pooled plasma and urine samples in an approximately 6000 bp genomic region with stable nucleosomes (Chromosome 12p11.1). The vertical grey lines depict the local maxima of each peak for the pooled urine samples. Mean smoothed physical sequencing coverage calculated by centering all peaks at the local maxima in **FIG. 2B. FIG. 2C** depicts the percentage of nucleosome calls overlapping in pairwise comparisons of nucleosome positioning maps. Each comparison is between two plasma maps (CH01-BH01, CH01-IH01, CH01-IH02, CH01-HP), two urine maps (HU-CU1, HU-CU2, CU1-CU2) or between a plasma and a urine map (CH01-HU, CH01-CU1, CH01-CU2). The distribution of distances between adjacent peak centers (interpeak distance) in each nucleosome positioning map is shown in **FIG. 2D. FIG. 2E** illustrates the distribution of distances between nearest peaks in pairwise comparison of two nucleosome positioning maps. The distribution of distances between corresponding peak centers are shown. FIGs 2F-2G show the comparison of plasma and urine median interpeak distance in 500 kb bins annotated as compartment B (closed chromatin regions) and compartment A (open chromatin regions) from Hi-C chromatin contact map of a lymphoblastoid cell line (GM12878). FIGs 2H-2I show the comparison of plasma and urine mean fragment size in 500 kb bins annotated as compartment A and B.

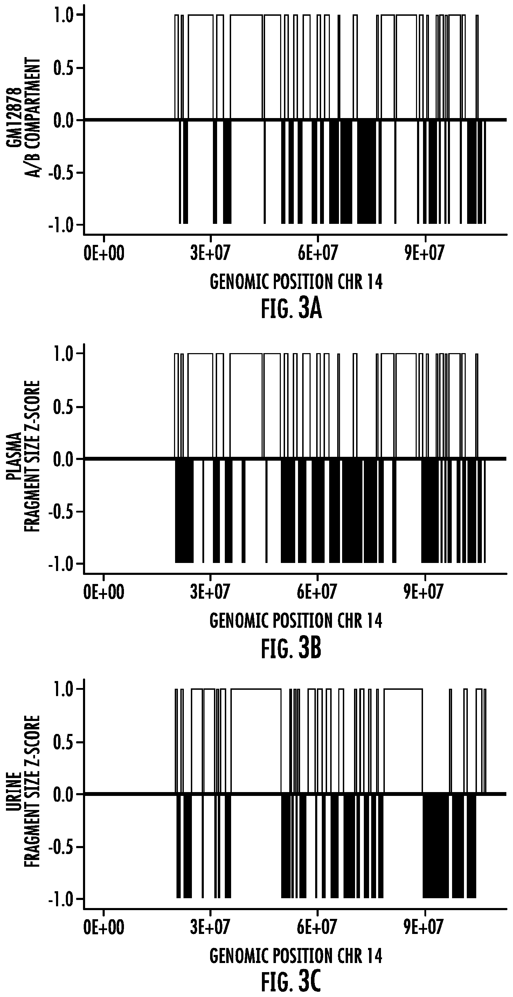

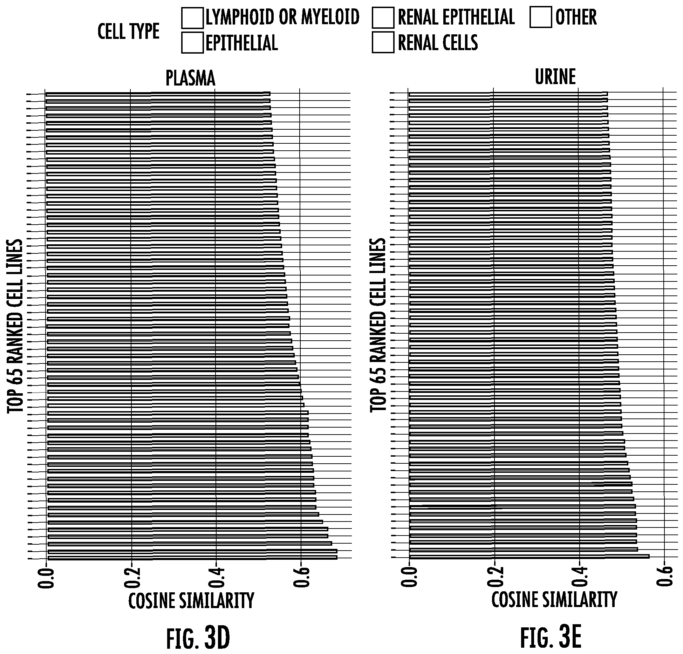

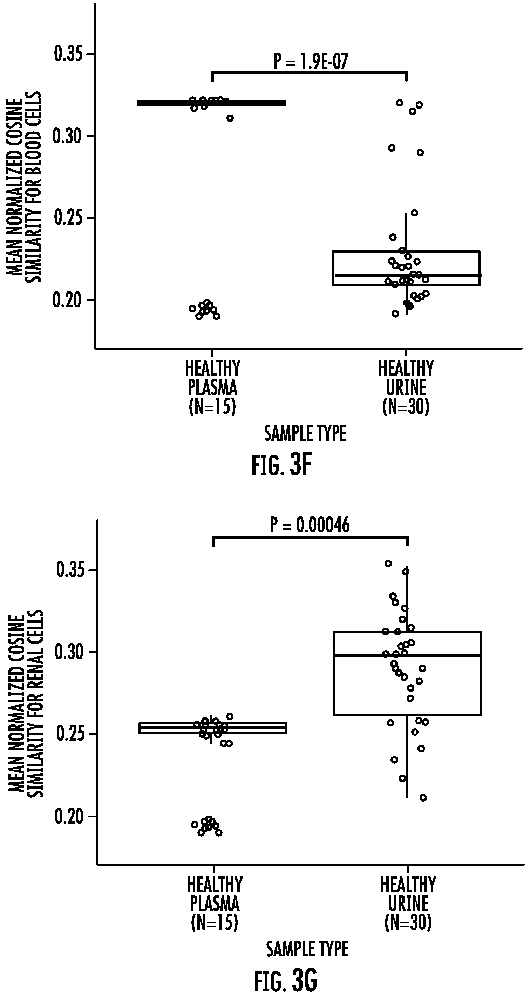

FIGs 3A-3G depict the comparison of cfDNA fragment size with chromatin accessibility across cell types. **FIG. 3A** shows the distribution of A (open chromatin) and B (closed chromatin) compartments in non-overlapping 500 kb bins on chromosome 14 from Hi-C chromatin contact map of lymphoblastoid cell lines (GM12878). A and B compartments are shown in red and blue colors respectively. **FIGs 3B-3C** depict the distribution of median cfDNA fragment size in corresponding 500 kb bins, normalized to a z-score for pooled plasma and urine samples respectively. Bins with negative and positive z-score values were transformed to -1 and 1 and colored red and blue, respectively. **FIGs 3D-3E** show 65 cell lines or tissues with highest cosine similarity between cfDNA fragment size and DHS sites in 500 kb bins across the genome. **FIG. 3F** depicts the comparison of mean quantile normalized cosine similarity scores for bone marrow, lymphoid or myeloid cell lines (n=24) in individual plasma and urine samples. Comparison of mean quantile normalized cosine similarity scores for renal tissues and renal epithelial cell lines (n=4) for individual plasma and urine samples is shown in **FIG. 3G.**

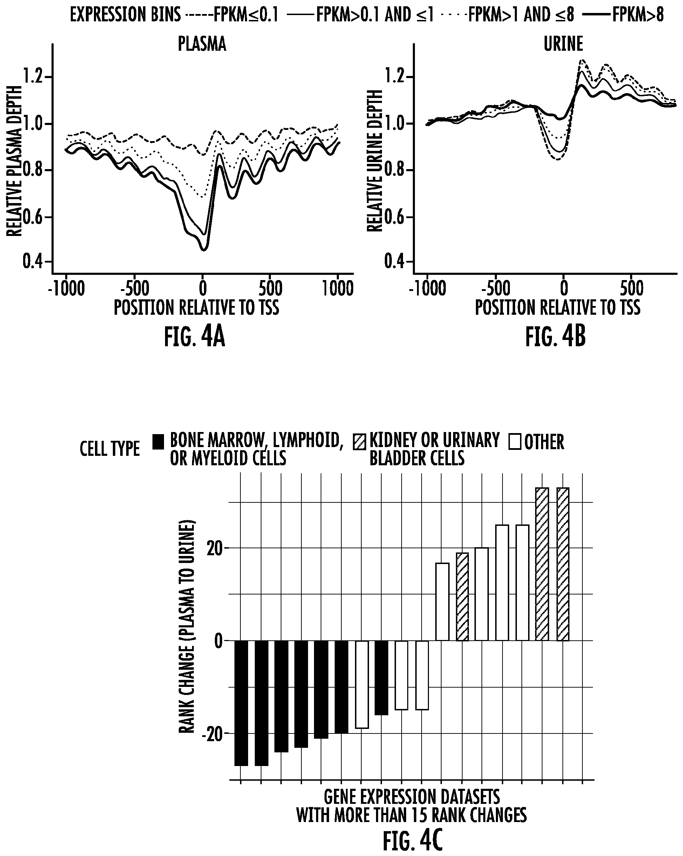

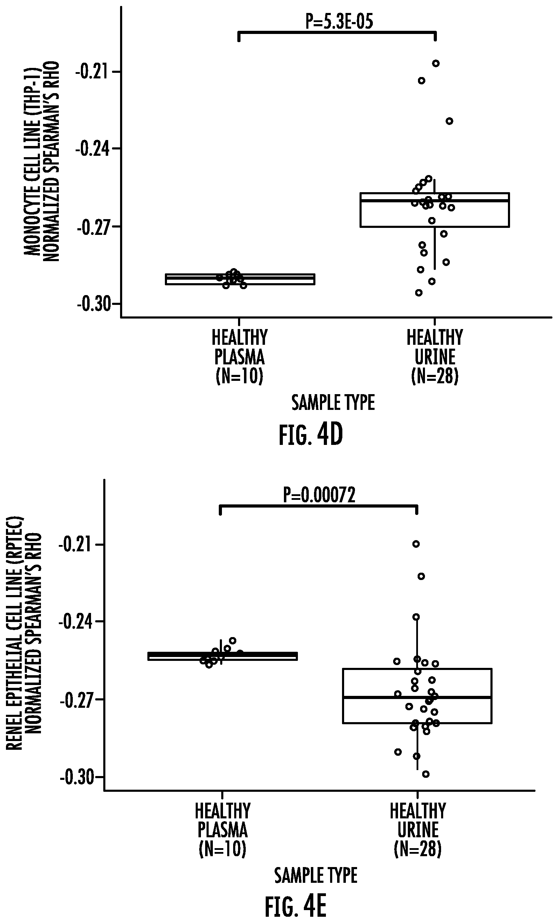

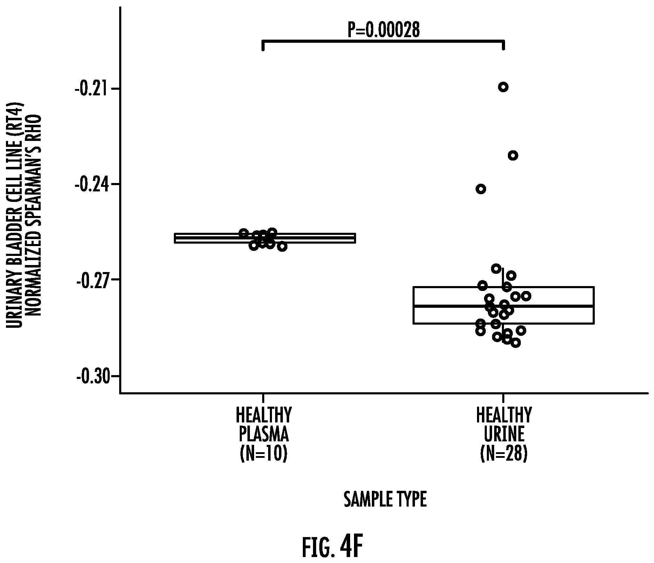

**FIGs 4A-4F** depict the comparison of cfDNA coverage at transcription start sites and correlation to gene expression across cell types. **FIGs 4A-4B** illustrate mean pooled plasma and urine sequencing depth at the transcription start sites (TSS) of genes binned by their expression levels in fragments per kilobase of transcript per million mapped reads (FPKM). Gene expression levels in plasma were used for this analysis. A depletion of coverage is observed at transcription start sites in plasma **(FIG. 4A)** and in urine samples **(FIG. 4B)**. Such depletion is greatest for genes with the highest expression. **FIG. 4C** depicts rank changes in correlation between sequencing coverage in the nucleosome depleted region and gene expression across plasma and urine cfDNA. Cell lines that changed by at least 15 ranks are shown here. **FIGs 4D-4F** depict the comparison of mean quantile normalized Spearman's rho for gene expression data from a monocyte cell line (THP-1), renal epithelial cell line (RPTEC), and urinary bladder cell line (RT4) in individual plasma and urine samples.

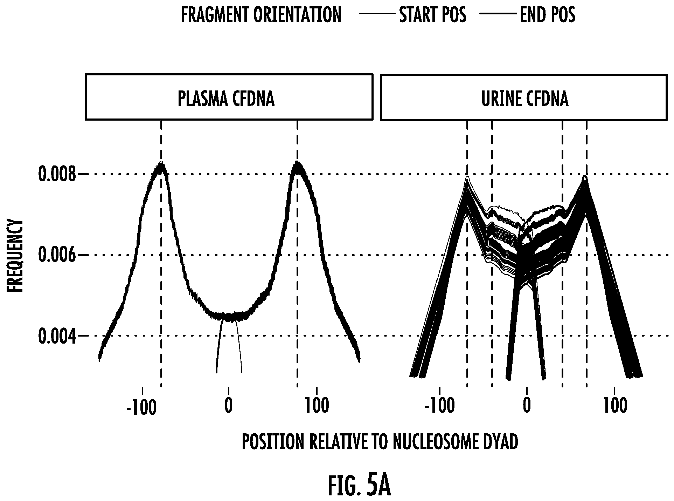

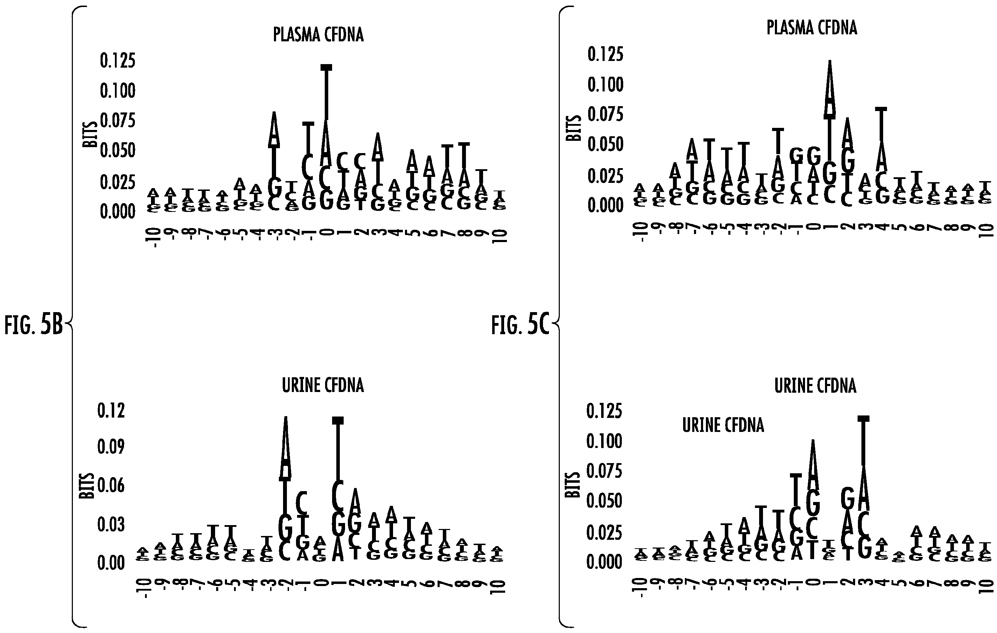

**FIGs 5A-5C** depict the characterization of cell-free DNA fragment end sites. **FIG. 5A** illustrates genome-wide distribution of fragment start and end sites of individual plasma and urine samples relative to nucleosome dyads. Comparison was made with a published plasma-based nucleosome positioning map (CH01) for plasma cfDNA samples and a urine-based nucleosome positioning map (HU) for urine cfDNA samples. The vertical lines are drawn at 77 bp downstream and upstream from the nucleosome dyad for the plasma cfDNA distribution and at 67 bp and 40 bp downstream and upstream from the dyad for the urine cfDNA distribution. **FIGs 5B-5C** show nucleotide frequencies surrounding 10 bp upstream and downstream of fragment starts **(FIG. 5B)** and ends **(FIG. 5C)** in pooled plasma and urine cfDNA samples.

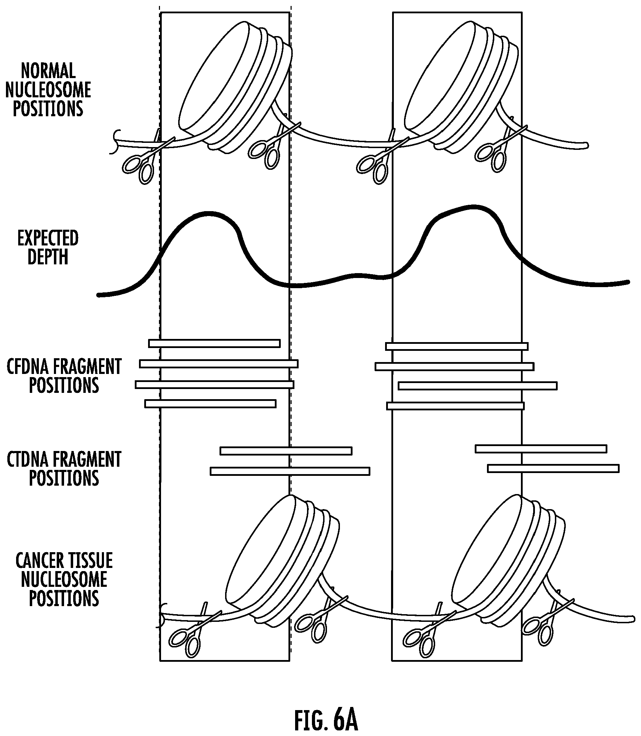

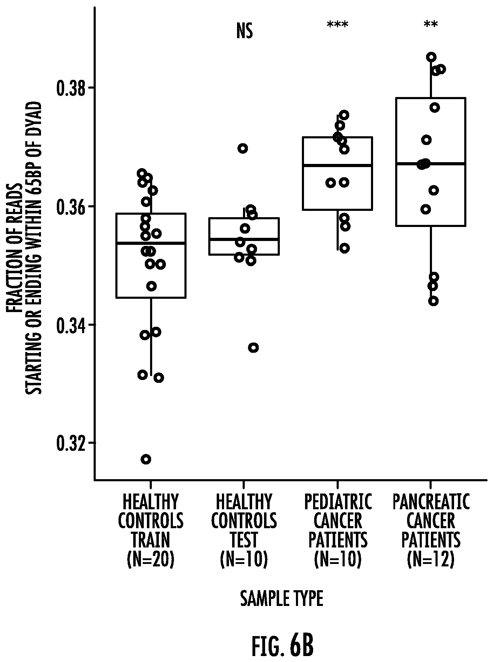

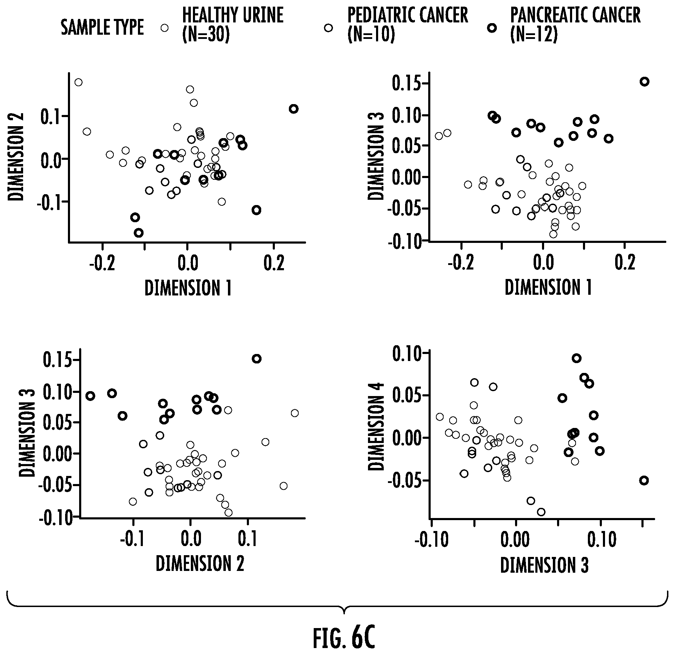

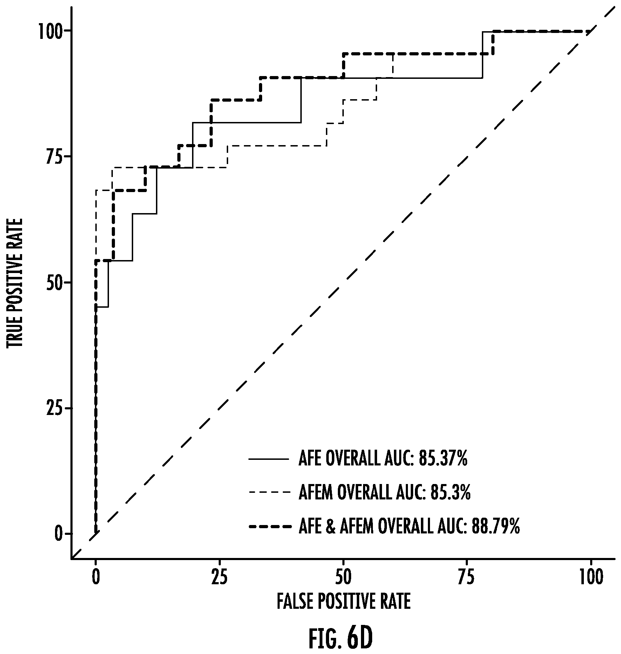

**FIGs 6A-6D** illustrate the evaluation of aberrant cfDNA fragments in urine from cancer patients. **FIG. 6A** shows a schematic representation of aberrant cfDNA fragments in urine samples from cancer patients. The illustration shows DNA wrapped in nucleosomes. Fragment start and end positions flank regions protected by nucleosomes and are clustered away from nucleosome centers. In patients with cancer, differences in nucleosome positioning in cancer cells that contribute cfDNA into urine may lead to a higher abundance of fragment start and end sites in unexpected genomic regions (such as regions protected by nucleosome in healthy control samples). **FIG. 6B** depicts the fraction of urine cfDNA reads starting or ending within 65 bp of nucleosome dyads in reference nucleosome occupancy based on pooled urine cfDNA data from 20 controls (training set). The fractions from training set are compared to urine samples from 10 additional controls (test set), 10 pediatric cancer patients, and 12 pancreatic cancer patients. The ns, **, and *** represent p-values >0.05, <0.01, and <0.0001, respectively. **FIG. 6C** shows a multidimensional scaling (MDS) analysis of nucleotide frequencies in 10 bp region surrounding urine cfDNA fragment start and end sites and **FIG. 6D** depicts a ROC analysis for classifying urine samples from controls and cancer patients using aberrant fraction ends (AFE), aberrant fraction end motifs (AFEM) or both. For AFE analysis, the fractions shown in **FIG. 6A** was used for ROC analysis. For AFEM and for the combination of AFE and AFEM, probabilities from a logistic regression fit to the first 4 MDS dimensions and AFE was used for ROC analysis.

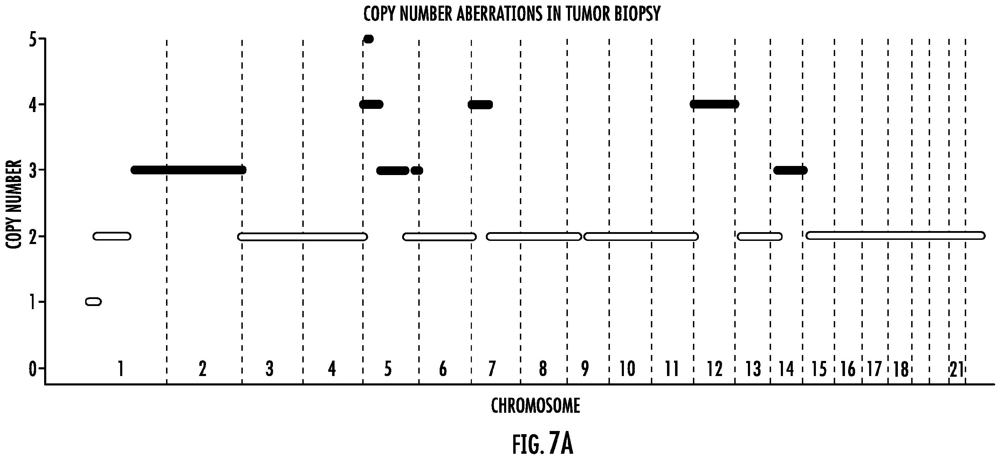

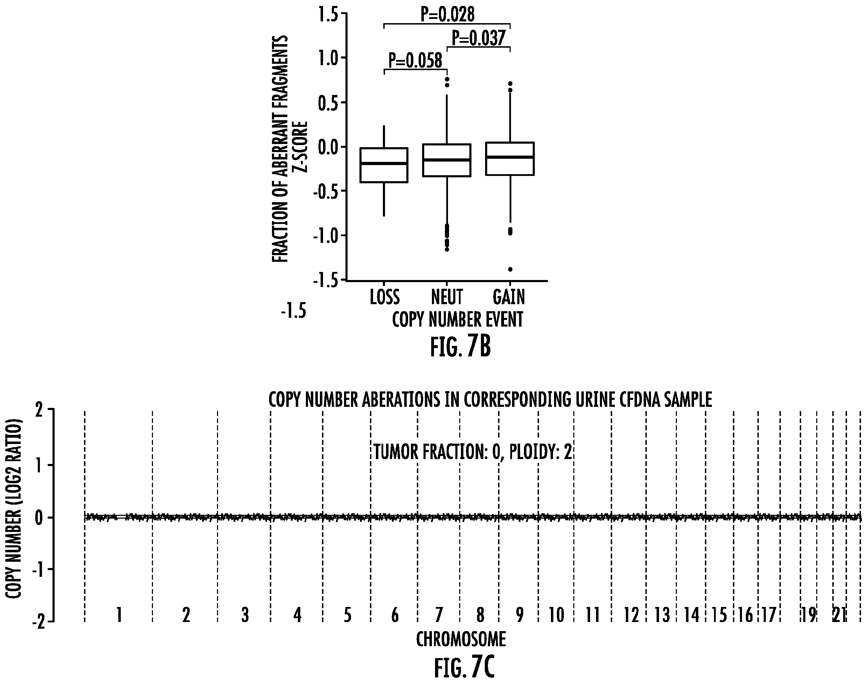

**FIGs 7A-7C** depict a comparison of fraction of aberrant fragments in urine cfDNA with copy number aberrations in the tumor and urine. Copy number aberrations observed in tumor DNA from a patient with rhabdomyosarcoma are shown in **FIG. 7A.** A copy number of 1 indicates a loss in copy numbers, a copy number of 2 means there was no copy number change, and a copy number of 3 or more indicates a gain in copy number. **FIG. 7B** shows the fraction of aberrant cfDNA fragments in a corresponding urine sample with comparison of copy number gain, neutral and loss regions. P-values are indicated, showing significant differences between gain vs loss and gain vs. neutral regions. **FIG. 7C** indicates that copy number aberrations were not observed directly in urine cfDNA by read density analysis and a tumor fraction of zero was observed (below limit of detection).

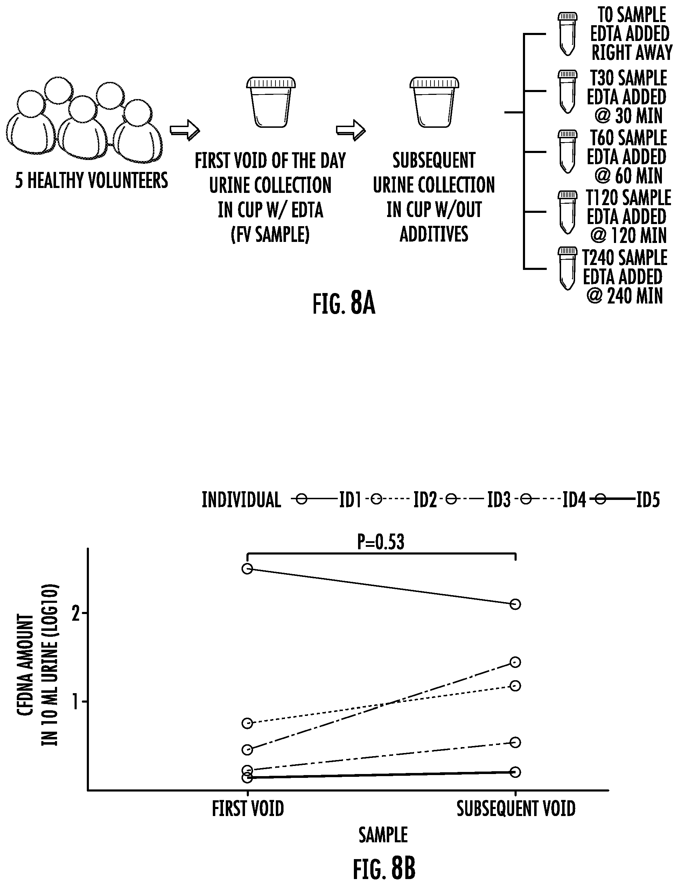

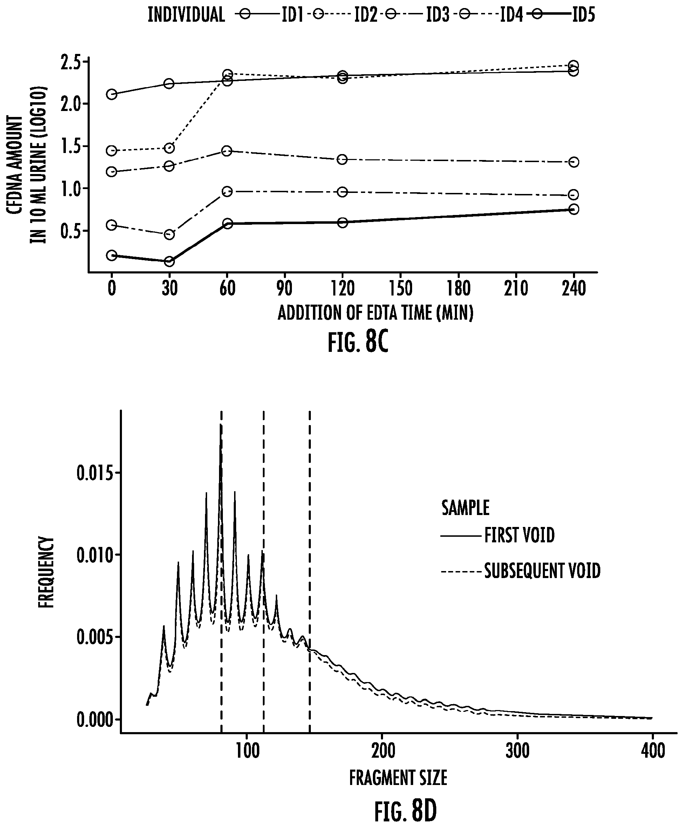

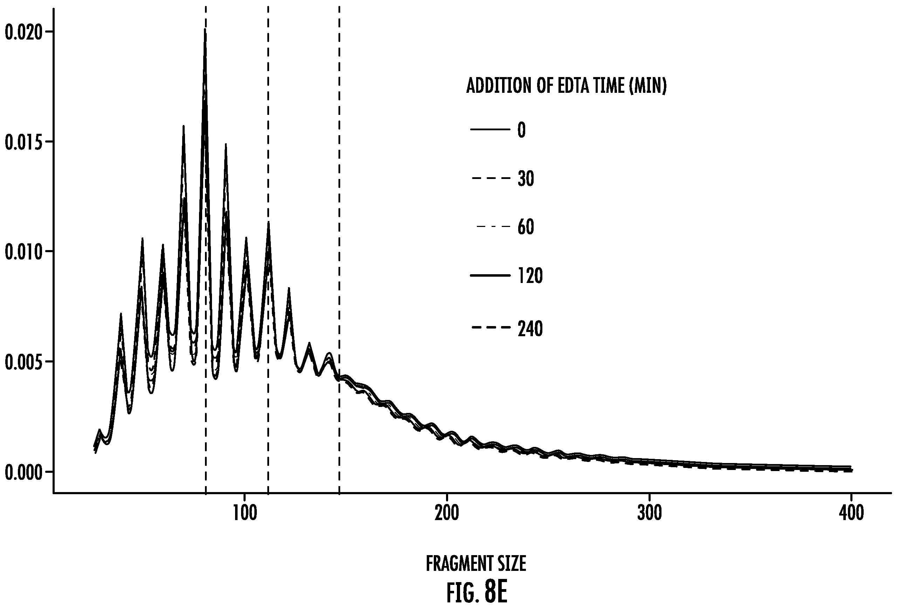

**FIGs 8A-8E** depicts the pre-analytical variation in urine cfDNA fragmentation patterns. **FIG. 8A** illustrates a schematic representation of the experiment design. Paired urine samples were collected from 5 healthy individuals, including first void of the day and a subsequent sample. The subsequent sample was processed in 5 different aliquots with increasing delays in processing. **FIG. 8B** shows comparison of cfDNA yield between the first void sample (FV) and the subsequent sample (T0). cfDNA yield was measured using fluorometry. No significant difference was observed. **FIG. 8C** shows the comparison of cfDNA yield between 5 aliquots of the subsequent sample. cfDNA yield was measured using fluorometry. An increase in total cfDNA yield was observed when sample processing was delayed by 60 minutes or longer. **FIG. 8D** illustrates the comparison of cfDNA fragment size distributions between first void (FV) and subsequent sample (T0). **FIG. 8E** depicts the comparison of cfDNA fragment size distributions between 5 aliquots of the subsequent sample.

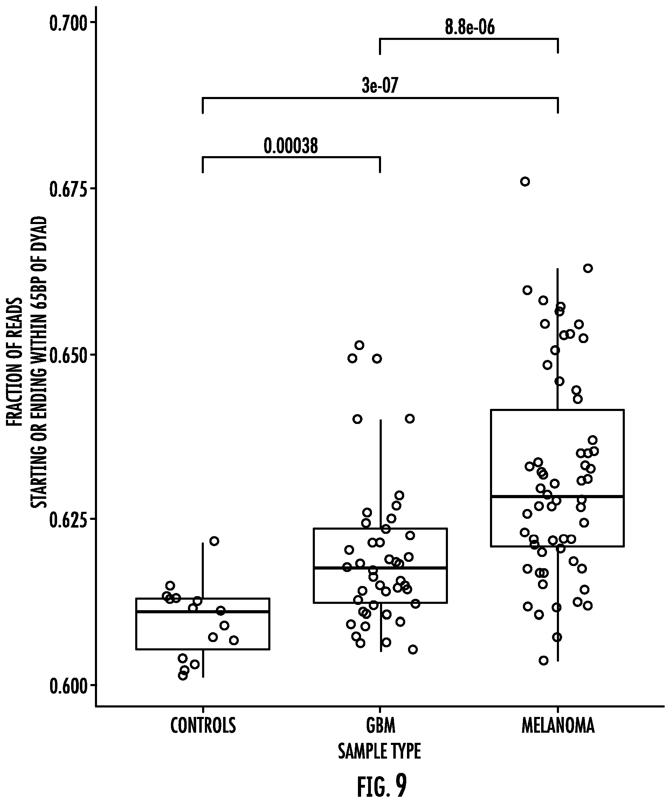

**FIG. 9** depicts the fraction of cfDNA aberrant ends in unexpected positions in plasma samples from healthy controls, patients with glioblastoma (GBM) and patients with metastatic cancer.

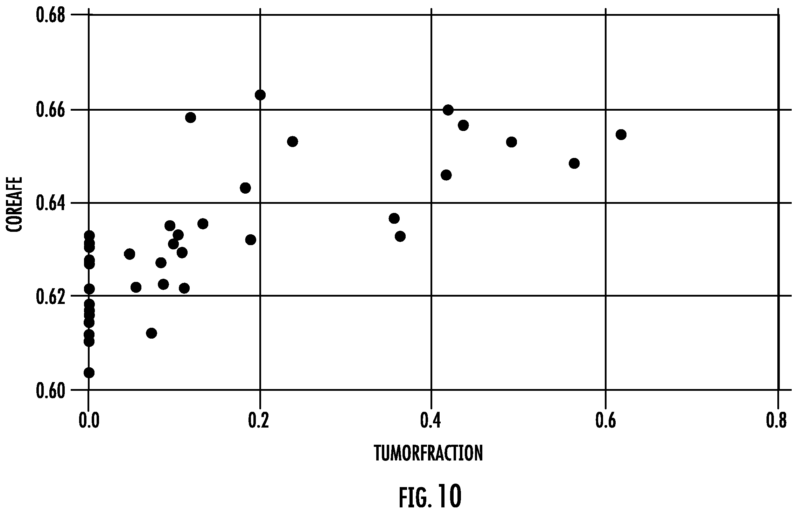

**FIG. 10** depicts the association between fractions of aberrant fragment ends and tumor fractions in plasma measured using sWGS copy number analysis.

## DETAILED DESCRIPTION

As used herein and in the appended claims, the singular forms "a," "an," and "the" include plural referents unless the context clearly dictates otherwise. For example, "a" or "an" means "at least one" or "one or more." Thus, reference to "an abnormal cell type" refers to one or more abnormal cells or types of abnormal cells.

Throughout this disclosure, various aspects of the claimed patient matter are presented in a range format. It should be understood that the description in range format is merely for convenience and brevity and should not be construed as an inflexible limitation on the scope of the claimed patient matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed patient matter. The upper and lower limits of these smaller ranges may independently be included in the smaller ranges, and are also encompassed within the claimed patient matter, patient to any specifically excluded limit in the stated range. Where the stated range includes one or both of the limits, ranges excluding either or both of those included limits are also included in the claimed patient matter. This applies regardless of the breadth of the range.

A "patient" as used herein refers to an organism, or a part or component of the organism, to which the provided methods, apparatuses, and systems can be administered or applied. For example, the patient can be a mammal or a cell, a tissue, an organ, or a part of the mammal. Mammals include, but are not limited to, humans, and non-human animals, including farm animals, sport animals, rodents and pets.

The terms "nucleic acid," "nucleotide," "polynucleotide," and "oligonucleotide" are used interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The frequency of a nucleotide refers to a percentage of the number of times a given nucleotide is found in a region or sequence of nucleic acids out of the total number of nucleotides in the region or sequence.

The term "biological sample" refers to a body sample from any animal, but preferably is from a mammal, more preferably from a human. Such samples include biological fluids such as serum, plasma, vitreous fluid, lymph fluid, synovial fluid, follicular fluid, seminal fluid, amniotic fluid, milk, whole blood, urine, cerebro-spinal fluid, saliva, sputum, tears, perspiration, mucus, and tissue culture medium, as well as tissue extracts such as homogenized tissue, and cellular extracts.

The term "control" refers to a reference sample to which a biological sample is compared against. The control sample may be used to set a threshold for distribution of start and end sites in cfDNA fragments, nucleotide frequency at start and end sites, the aberrant end fraction, a nucleosome map and the position of nucleosome peaks, the median fragment length, and/or the coverage at transcriptional start sites. The sample may be any biological sample, and preferably is the same type of biological samples as the sample that is being compared against the control. A control is preferably obtained from the same species as the sample that is being compared to the control. This is preferably from a mammal, and more preferably from a human. The control sample obtained from one subject or may be generated by pooling samples from multiple subjects. These subjects may be healthy. In other instances, the control is a subject that exhibits the same disease or disease subtype, including for example cancer. In yet other instances, the control is a sample obtained from the subject, e.g., earlier or later than the sample being tested.

The term "abnormal cell" or "unexpected cell" refers to a cell that would not normally be found in a healthy patient, or a cell that is not present in a control sample. Such cells include cancer cells, tumors, and cells that are infected with a pathogen. Abnormal cells may also include an unusually high occurrence of damaged or dead cells. As these cells die or are damaged, they release cfDNA that may be detected in a biological sample.

The term "aberrant end fraction" refers to the fraction of cfDNA fragments that contain unexpected end sequences. The repositioning of nucleosomes in cancer cells will produce cfDNA fragments that exhibit a higher abundance of fragment start and end sites in unexpected genomic regions. These unexpected genomic regions may include regions that are normally protected by nucleosomes in healthy control samples.

Plasma is a conventional biological sample that is regularly used in various testing modalities. cfDNA is readily detected in plasma in relatively stable concentrations, and plasma cfDNA exhibits mostly uniform fragment sizes. The positioning of plasma cfDNA and protection from degradation captures the footprint of contributing tissues being informative biomarkers of disease. While urine is a non-invasive sample that could easily be collected at home, in large volumes, and without the need for technicians like a phlebotomist, deficiencies in our understanding of urine cfDNA and difficulties in analyzing urine cfDNA have prevented its use as a sample source for noninvasive diagnostics. Urine cfDNA is generally derived from two sources: degraded DNA from the urinary tract and transrenal DNA that is excreted from plasma. In contrast to plasma cfDNA, concentrations of urine cfDNA are not stable and can vary from sample to sample, requiring sensitive methods to detect and analyze the cfDNA fragments. Additionally, urine cfDNA fragments are generally smaller in size, more variable in size, and more degraded relative to plasma cfDNA fragments, increasing the difficulty of analyzing urine cfDNA fragments using traditional methods.

To address this gap and improve detection and analysis of cfDNA from non-invasive samples, such as, urine, a new method is disclosed herein that utilizes whole genome sequencing (WGS) to detect cfDNA fragmentation profiles and then analyzes cfDNA fragmentation profile patterns to identify cfDNA from unexpected or abnormal cell types, including cancer cells, that indicate the presence of a disease. This method can also be used to distinguish cancer subtypes and to monitor a patient's response to treatment, thereby improving the ability to personalize treatments based on the cancer subtype detected and the response to treatment.

In certain aspects, the disclosure provides a method of detection of a cfDNA profile from a sample, the method comprising: a) obtaining a sample from the subject; b) extracting cfDNA from the sample to obtain cfDNA fragments; c) performing WGS on the cfDNA fragments extracted from the sample to generate sequencing reads for the cfDNA fragments; d) determining from the sequencing reads a fragmentation profile of the cfDNA fragments.

In other aspects, the disclosure provides a method of detection of disease in a subject through sequencing and analysis of cfDNA, the method comprising: a) obtaining a sample from the subject; b) extracting cfDNA from the sample to obtain cfDNA fragments; c) performing WGS on the cfDNA fragments extracted from the sample to generate sequencing reads for the cfDNA fragments; d) comparing the fragmentation pattern of the cfDNA fragments to a fragmentation pattern of cfDNA from a control sample; and e) determining the presence of a disease in the subject based on the fragmentation pattern of the cfDNA fragments deviating from the fragmentation pattern of the control sample.

In yet other aspects, the disclosure provides a method of detection of disease in a subject through sequencing and analysis of cfDNA, the method comprising: a) obtaining a plurality of control samples each from healthy subjects; b) extracting cfDNA from the control samples to obtain control cfDNA fragments; c) performing whole genome sequencing on the control cfDNA fragments to generate sequencing reads for the control cfDNA fragments; d) pooling the sequencing reads for the control cfDNA fragments to determine a pooled control fragmentation pattern; and e) determining presence of the disease based on the fragmentation pattern of the cfDNA fragments obtained from the sample obtained from a subject that deviate from the pooled control fragmentation pattern.

In one embodiment, the fragmentation pattern of the cfDNA fragments is based on a comparison of the distribution of start sites and end sites of the cfDNA fragments in the subjects cfDNA sample to the control cfDNA sample. In certain embodiments, a sample's deviation in the distribution of start and end sites in comparison to the control sample indicate the presence of disease and/or the contribution of cfDNA from unexpected or abnormal cells. In another embodiment the fragmentation pattern of cfDNA fragments is based on the nucleotide frequency at start sites and end sites of the cfDNA fragments. A threshold for nucleotide frequency at start sites and end sites of the cfDNA fragments is determined based on the nucleotide frequency of the control sample or another reference sample. The presence of an abnormal cell type is identified by the nucleotide frequency in the cfDNA fragments of the subject's sample exceeding or falling below the nucleotide frequency of the reference sample.

In other embodiments, the fragmentation pattern of the cfDNA fragments is characterized by determining the aberrant ends fraction of the cfDNA fragments. The repositioning of nucleosomes in cancer cells will ultimately produce cfDNA fragments that exhibit a higher abundance of fragment start and end sites in unexpected genomic regions. These unexpected genomic regions includes regions that are normally protected by nucleosomes in healthy control samples. The fraction of cfDNA fragments containing aberrant ends is referred to as the aberrant ends fraction. The presence of an abnormal cell type or a disease is identified by the aberrant ends fraction in the cfDNA profile of the subject's sample deviating from the aberrant ends fraction of the cfDNA profile of the control sample.

In another embodiment, the fragmentation pattern of cfDNA fragments is used to generate a nucleosome map that identifies the position of nucleosomes in the sample. The nucleosome map displays positions of nucleosome peaks, indicating open and closed chromatin regions in the subject's genome. Open chromatin regions indicate regions of the genome that do not contain nucleosomes. These open regions are able to be bound by various protein factors and regulatory elements and transcribed. Closed chromatin regions are regions of the genome that surround nucleosomes and are inaccessible to protein factors, regulatory elements, and other molecules. These closed chromatin regions are not able to be transcribed. Thus, the positioning of these open and closed chromatin regions as indicated by sample cfDNA fragments identifies the type of cell or state of the cell that a cfDNA fragment came from. The fragmentation patterns from the control sample is used to create a reference nucleosome map. The nucleosome map generated from cfDNA fragments from the sample of the patient identifies nucleosome peaks and is compared to the reference nucleosome map. In some embodiments, the position of nucleosome peaks in the sample from the patient is compared to the position of nucleosome peaks in the control sample. Deviation in peak position in the fragments from the subject sample and control sample can indicate the presence of disease or that the cfDNA in the sample came from an abnormal or unexpected cell type.

In another embodiment, the fragmentation pattern of cfDNA fragments is determined by the determination of median fragment length of the cfDNA fragments. In certain specific embodiments, deviations in cfDNA fragment length between the control sample and the subject's sample indicate the presence of disease. In certain aspects, the median fragment length of the cfDNA fragments is also compared to chromatin states of a selected cell type to identify cellular sources of cfDNA that indicative of the type of disease a subject has.

In an embodiment, the fragmentation pattern of the cfDNA fragments is characterized by determining the coverage of cfDNA fragments at transcription start sites (TSS). The coverage of TSS indicates specific genes that are being transcribed in the cell that the cfDNA fragments come from. The coverage of TSS in a subject's cfDNA profile is compared to the coverage of TSS in the control cfDNA profile and deviations in the TSS coverage indicates the presence of disease or that the cfDNA fragments in a sample are from abnormal or unexpected cell types. The coverage at TSS of cfDNA fragments is compared with gene expression in a selected cell type to determine the cellular source of a cfDNA fragment.

In another embodiment, the presence of an abnormal cell type identified by the fragmentation pattern of the cfDNA in the subject's sample indicates the presence of disease in the subject. In another embodiment, disease is detected by comparing the contribution of cfDNA from abnormal cells in the sample from a subject based on cfDNA fragmentation patterns to the control sample. In one embodiment, the disease is cancer. In an embodiment, a treatment is administered to the subject based on the presence of disease as indicated by deviations in cfDNA fragmentation patterns.

In one embodiment, the disclosure provides a method of monitoring treatment response in a subject through sequencing and analysis of cfDNA, the method comprising: a) obtaining a first sample from the subject prior to administering a treatment to a subject; b) obtaining a second sample from the subject after administering treatment to the subject; c) extracting cell-free DNA (cfDNA) from the first sample to obtain a first set of cfDNA fragments; d) extracting cell-free DNA (cfDNA) from the second sample to obtain a second set of cfDNA fragments; e) performing whole genome sequencing on the first and second sets of cfDNA fragments extracted from the sample to generate sequencing reads for the first and second sets of cfDNA fragments; f) determining from the sequencing reads a first fragmentation pattern of the first cfDNA fragments and a second fragmentation pattern of the second cfDNA fragments; g) comparing the second fragmentation pattern to the first fragmentation pattern; and h) determining a status of the treatment response in the subject based on the second fragmentation pattern deviating from the first fragmentation pattern.

In one embodiment, the disclosure provides a method of monitoring treatment response in a subject through sequencing and analysis of cfDNA, the method comprising: a) obtaining a first sample from the subject prior to administering a treatment to a subject; b) obtaining a second sample from the subject after administering treatment to the subject; c) extracting cell-free DNA (cfDNA) from the first sample to obtain a first set of cfDNA fragments; d) extracting cell-free DNA (cfDNA) from the second sample to obtain a second set of cfDNA fragments; e) performing whole genome sequencing on the first and second sets of cfDNA fragments extracted from the sample to generate sequencing reads for the first and second sets of cfDNA fragments; f) determining from the sequencing reads a first fragmentation pattern of the first cfDNA fragments and a second fragmentation pattern of the second cfDNA fragments; g) comparing the second fragmentation pattern to the first fragmentation pattern; and h) determining a status of the treatment response in the subject based on the presence or absence of cfDNA from an abnormal cell type in the second sample compared to the first sample.

In certain embodiments, the disclosure provides a method of determination of a cancer subtype in a subject through sequencing and analysis of cfDNA, the method comprising: a) obtaining a sample from the subject; b) extracting cfDNA from the sample to obtain cfDNA fragments; c) performing WGS on the cfDNA fragments extracted from the sample to generate sequencing reads for the cfDNA fragments; d) determining from the sequencing reads a fragmentation pattern of the cfDNA fragments; e) comparing the fragmentation pattern of the cfDNA fragments from the subject's sample to a fragmentation pattern of a reference sample; and f) determining the cancer subtype based on a similarity or difference in the fragmentation pattern of the cfDNA fragments from the subject's sample to the fragmentation pattern of the reference sample. In one embodiment the reference sample is from a healthy control subject. In another embodiment, the reference sample is from a subject having a similar cancer subtype.

In other embodiments, the disclosure provides a method of determination of a cancer subtype in a subject through sequencing and analysis of cfDNA, the method comprising: a) obtaining a plurality of reference samples; b) extracting cfDNA from the reference samples to obtain reference cfDNA fragments; c) performing WGS on the reference cfDNA fragments to generate sequencing reads for the reference cfDNA fragments; d) pooling the sequencing reads for the reference cfDNA fragments to determine a pooled reference fragmentation pattern; and e) determining the cancer subtype based on the fragmentation pattern of the cfDNA fragments deviating from the pooled reference fragmentation pattern. In one embodiment the reference samples are each obtained from healthy subjects. In another embodiment the reference samples are each obtained from subjects having the same cancer subtype.

## Sample Preparation

The methods of this disclosure may have a wide variety of uses in the manipulation, preparation, identification and/or quantification of cell free polynucleotides. Examples of polynucleotides include but are not limited to: DNA, RNA, amplicons, cDNA, dsDNA, ssDNA, plasmid DNA, cosmid DNA, high Molecular Weight (MW) DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA (e.g., retroviral RNA).

Cell free polynucleotides may be derived from a variety of sources including human, mammal, non-human mammal, ape, monkey, chimpanzee, reptilian, amphibian, or avian, sources. Further, samples may be extracted from variety of animal fluids containing cell free sequences, including but not limited to blood, serum, plasma, vitreous, sputum, urine, tears, perspiration, saliva, semen, mucosal excretions, mucus, spinal fluid, amniotic fluid, lymph fluid and the like. Cell free polynucleotides may be fetal in origin (via fluid taken from a pregnant patient), or may be derived from tissue of the patient itself.

Isolation and extraction of cell free polynucleotides may be performed through collection of bodily fluids using a variety of techniques. In some cases, collection may comprise aspiration of a bodily fluid from a patient using a syringe. In other cases, collection may comprise pipetting or direct collection of fluid into a collecting vessel.

After collection of bodily fluid, cell free polynucleotides may be isolated and extracted using a variety of techniques known in the art. In some cases, cell free DNA may be isolated, extracted and prepared using commercially available kits such as the Qiagen Qiamp® Circulating Nucleic Acid Kit protocol. In other examples, Qiagen Qubit™ dsDNA HS Assay kit protocol, Agilent™ DNA 1000 kit, or TruSeq™ Sequencing Library Preparation; Low-Throughput (LT) protocol may be used.

Generally, cell free polynucleotides are extracted and isolated by from bodily fluids through a partitioning step in which cell free DNAs, as found in solution, are separated from cells and other non-soluble components of the bodily fluid. Partitioning may include, but is not limited to, techniques such as centrifugation or filtration. In other cases, cells are not partitioned from cell free DNA first, but rather lysed. In this example, the genomic DNA of intact cells is partitioned through selective precipitation. Cell free polynucleotides, including DNA, may remain soluble and may be separated from insoluble genomic DNA and extracted. Generally, after addition of buffers and other wash steps specific to different kits, DNA may be precipitated using isopropanol precipitation. Further clean up steps may be used such as silica-based columns to remove contaminants or salts. General steps may be optimized for specific applications. Nonspecific bulk carrier polynucleotides, for example, may be added throughout the reaction to optimize certain aspects of the procedure such as yield.

Isolation and purification of cell free DNA may be accomplished using any means, including, but not limited to, the use of commercial kits and protocols provided by companies such as Sigma Aldrich, Life Technologies, Promega, Affymetrix, IBI or the like. Kits and protocols may also be non-commercially available.

After isolation, in some cases, the cell free polynucleotides are pre-mixed with one or more additional materials, such as one or more reagents (e.g., ligase, protease, polymerase) prior to sequencing.

The methods of this disclosure may also enable the cell free polynucleotides to be tagged or tracked in order to permit subsequent identification and origin of the particular polynucleotide. This feature is in contrast with other methods that use pooled or multiplex reactions and that only provide measurements or analyses as an average of multiple samples. Here, the assignment of an identifier to individual or subgroups of polynucleotides may allow for a unique identity to be assigned to individual sequences or fragments of sequences. This may allow acquisition of data from individual samples and is not limited to averages of samples.

In some examples, nucleic acids or other molecules derived from a single strand may share a common tag or identifier and therefore may be later identified as being derived from that strand. Similarly, all of the fragments from a single strand of nucleic acid may be tagged with the same identifier or tag, thereby permitting subsequent identification of fragments from the parent strand. In still other cases, the systems and methods can be used as a PCR amplification control. In such cases, multiple amplification products from a PCR reaction can be tagged with the same tag or identifier. If the products are later sequenced and demonstrate sequence differences, differences among products with the same identifier can then be attributed to PCR error.

Additionally, individual sequences may be identified based upon characteristics of sequence data for the read themselves. For example, the detection of unique sequence data at the beginning (start) and end (stop) portions of individual sequencing reads may be used, alone or in combination, with the length, or number of base pairs of each sequence read unique sequence to assign unique identities to individual molecules. Fragments from a single strand of nucleic acid, having been assigned a unique identity, may thereby permit subsequent identification of fragments from the parent strand. This can be used in conjunction with bottlenecking the initial starting genetic material to limit diversity.

Further, using unique sequence data at the beginning (start) and end (stop) portions of individual sequencing reads and sequencing read length may be used, alone or combination, with the use of barcodes. In some cases, the barcodes may be unique as described herein. In other cases, the barcodes themselves may not be unique. In this case, the use of non-unique barcodes, in combination with sequence data at the beginning (start) and end (stop) portions of individual sequencing reads and sequencing read length may allow for the assignment of a unique identity to individual sequences. Similarly, fragments from a single strand of nucleic acid having been assigned a unique identity, may thereby permit subsequent identification of fragments from the parent strand.

Generally, the methods and systems provided herein are useful for preparation of cell free polynucleotide sequences to a down-stream application sequencing reaction. Often, a sequencing method is classic Sanger sequencing. Sequencing methods may include, but are not limited to: high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next generation sequencing, Single Molecule Sequencing by Synthesis (SMSS)(Helicos), massively-parallel sequencing, Clonal Single Molecule Array (Solexa), shotgun sequencing, Maxim-Gilbert sequencing, primer walking, and any other sequencing methods known in the art.

## Cancer Detection and Monitoring

Numerous cancers are detected and monitored using the methods described herein. Cancer cells, as most cells, can be characterized by a rate of turnover, in which old cells die and are replaced by newer cells. Generally dead cells, in contact with vasculature in a given patient, may release DNA or fragments of DNA into the bloodstream. This is also true of cancer cells during various stages of the disease. This phenomenon may be used to detect the presence or absence of cancers in individuals using the methods described herein.

For example, blood from patients at risk for cancer is drawn or urine is collected and the sample is prepared as described herein to generate a population of cell free polynucleotides. In one example, this might be cell free DNA. The methods of the disclosure employed to detect cfDNA fragment patterns and features that may be unique to certain cancers present. The method detects the presence of cancerous cells in the body, despite the absence of symptoms or other hallmarks of disease. The method also helps detect different subtypes of cancer based on the features of the cfDNA fragments detected in the patient sample.

The types and number of cancers that detected include but are not limited to blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like.

In an embodiment, the cancer is selected from the group consisting of oral cancer, prostate cancer, rectal cancer, non-small cell lung cancer, lip and oral cavity cancer, liver cancer, lung cancer, anal cancer, kidney cancer, vulvar cancer, breast cancer, oropharyngeal cancer, nasal cavity and paranasal sinus cancer, nasopharyngeal cancer, urethra cancer, small intestine cancer, bile duct cancer, bladder cancer, ovarian cancer, laryngeal cancer, hypopharyngeal cancer, gallbladder cancer, colon cancer, colorectal cancer, head and neck cancer, glioma, parathyroid cancer, penile cancer, vaginal cancer, thyroid cancer, pancreatic cancer, esophageal cancer, Hodgkin's lymphoma, leukemia-related disorders, mycosis fungoides, hematological cancer, hematological disease, hematological malignancy, minimal residual disease, and myelodysplastic syndrome.

In another embodiment, the cancer is selected from the group consisting of gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer, colorectal cancer, renal cancer, and bladder cancer.

In another embodiment, the cancer is non-small cell lung cancer, pancreatic cancer, breast cancer, ovarian cancer, colorectal cancer, or head and neck cancer. In yet another embodiment the cancer is a carcinoma, a tumor, a neoplasm, a lymphoma, a melanoma, a glioma, a sarcoma, or a blastoma.

In one embodiment, the carcinoma is selected from the group consisting of carcinoma, adenocarcinoma, adenoid cystic carcinoma, adenosquamous carcinoma, adrenocortical carcinoma, well differentiated carcinoma, squamous cell carcinoma, serous carcinoma, small cell carcinoma, invasive squamous cell carcinoma, large cell carcinoma, islet cell carcinoma, oat cell carcinoma, squamous carcinoma, undifferentiated carcinoma, verrucous carcinoma, renal cell carcinoma, papillary serous adenocarcinoma, merkel cell carcinoma, hepatocellular carcinoma, soft tissue carcinomas, bronchial gland carcinomas, capillary carcinoma, bartholin gland carcinoma, basal cell carcinoma, carcinosarcoma, papilloma/carcinoma, clear cell carcinoma, endometrioid adenocarcinoma, mesothelial carcinoma, metastatic carcinoma, mucoepidermoid carcinoma, cholangiocarcinoma, actinic keratoses, cystadenoma, and hepatic adenomatosis.

In another embodiment, the tumor is selected from the group consisting of astrocytic tumors, malignant mesothelial tumors, ovarian germ cell tumors, supratentorial primitive neuroectodermal tumors, Wilms tumors, pituitary tumors, extragonadal germ cell tumors, gastrinoma, germ cell tumors, gestational trophoblastic tumors, brain tumors, pineal and supratentorial primitive neuroectodermal tumors, pituitary tumors, somatostatin-secreting tumors, endodermal sinus tumors, carcinoids, central cerebral astrocytoma, glucagonoma, hepatic adenoma, insulinoma, medulloepithelioma, plasmacytoma, vipoma, and pheochromocytoma.

In yet another embodiment, the neoplasm is selected from the group consisting of intraepithelial neoplasia, multiple myeloma/plasma cell neoplasm, plasma cell neoplasm, interepithelial squamous cell neoplasia, endometrial hyperplasia, focal nodular hyperplasia, hemangioendothelioma, and malignant thymoma. In a further embodiment, the lymphoma may be selected from the group consisting of nervous system lymphoma, AIDS-related lymphoma, cutaneous T-cell lymphoma, non-Hodgkin's lymphoma, lymphoma, and Waldenstrom's macroglobulinemia. In another embodiment, the melanoma may be selected from the group consisting of acral lentiginous melanoma, superficial spreading melanoma, uveal melanoma, lentigo maligna melanomas, melanoma, intraocular melanoma, adenocarcinoma nodular melanoma, and hemangioma. In yet another embodiment, the sarcoma may be selected from the group consisting of adenomas, adenosarcoma, chondosarcoma, endometrial stromal sarcoma, Ewing's sarcoma, Kaposi's sarcoma, leiomyosarcoma, rhabdomyosarcoma, sarcoma, uterine sarcoma, osteosarcoma, and pseudosarcoma. In one embodiment, the glioma may be selected from the group consisting of glioma, brain stem glioma, and hypothalamic and visual pathway glioma. In another embodiment, the blastoma may be selected from the group consisting of pulmonary blastoma, pleuropulmonary blastoma, retinoblastoma, neuroblastoma, medulloblastoma, glioblastoma, and hemangioblastomas.

In certain embodiments, the methods provided herein are used to monitor already known cancers, or other diseases in a particular patient. This allows a practitioner to adapt treatment options in accord with the progress of the disease. In this example, the methods described herein track ctDNA in a particular patient over the course of the disease. In some instances, cancers progress, becoming more aggressive and genetically unstable. In other examples, cancers remain benign, inactive, dormant or in remission. The methods of this disclosure is useful in determining disease progression, remission or recurrence and the appropriate adjustments in treatment that are required for the disease state.

Further, the systems and methods described herein are useful in determining the efficacy of a particular treatment option. In one example, successful treatment options may actually increase the ctDNA detected in a patient's blood if the treatment is successful as more cancer cells die and shed DNA. In other examples, this does not occur. In another example, certain treatment options are correlated with genetic profiles of cancers over time. This correlation is useful in selecting a therapy. Additionally, if a cancer is observed to be in remission after treatment, the systems and methods described herein are useful in monitoring residual disease or recurrence of disease. Biological samples are collected longitudinally over time from a single patient and comparison of the cfDNA profiles in all of the different samples collected illustrates how the cancer or disease is progressing.

For example, the presence of cancer cells in the body is determined from DNA in a sample from a patient, e.g., a patient. The sample can be, e.g., cell free DNA or a tumor sample. A course of treatment is prescribed based on cfDNA fragment features that fall within a given frequency range including, e.g., their frequencies. A sample is taken from the patient at any subsequent time. cfDNA fragment features within the original range of frequency or a different range of frequency is determined. The course of treatment is then adjusted based on the subsequent measurements.

The present disclosure is further illustrated by the following examples that should not be construed as limiting.

## EXAMPLES

### Example 1. Materials and Methods

Patients and samples

For cancer patients, urine samples were collected at presentation and prior to treatment. Tumor samples analyzed were obtained at the time of diagnosis.

Sample processing and cfDNA quantification in urine and plasma

Urine samples were processed within 1 hour of collection. 0.8 ml of 0.5 M EDTA was added to 40 ml of urine, and 10 ml aliquots were centrifuged at 1,600g for 10 min and stored at - 80 °C. cfDNA was extracted from 10 ml urine using MagMAX Cell-Free DNA Isolation kit (Thermo Fisher Scientific) and eluted in 20-30 μl. Blood samples were collected in K2 EDTA BD Vacutainer tubes and processed within 2 hours of collection. Blood samples were centrifuged at 820g for 10 min at room temperature. 1 ml aliquots of plasma were further centrifuged at 16,000g for 10 min to pellet any remaining cellular debris. The supernatant was stored at -80 °C until DNA extraction. DNA was extracted using QIAamp Circulating Nucleic Acid kit (QIAGEN). DNA yield was measured using digital PCR assay[22]. In healthy volunteers, median urine cfDNA concentration was 0.82 ng/ml of urine (IQR: 2.3 ng/ml, n=30). Median plasma cfDNA concentration was 5.62 ng/ml of plasma (IQR: 4.75 ng/ml, n=16).

Sequencing library preparation

For plasma cfDNA samples, whole genome sequencing libraries were prepared using 1 ng input from healthy volunteer samples using ThruPLEX Tag-seq (Takara Bio). Sequencing was performed on a HiSeq 4000 (Illumina) to generate 75 bp paired-end reads. The library prep kit introduces a 6 bp unique molecular identifier and an 8-11 bp random stem on both ends of DNA fragments. These tags were removed using a custom Python script. For urine cfDNA samples, whole genome sequencing libraries were prepared using 0.6-67.3 ng input using ThruPLEX Plasma-seq (Takara Bio). Sequencing was performed on a NovaSeq 6000 (Illumina) to generate 110 bp paired-end reads.

Sequencing data and fragment size analysis

Sequencing data was de-multiplexed based on sample specific barcodes and converted to fastq files using Picard tools v2.2.1 and using Illumina bcl2fastq v2.20.0.422 for plasma and urine data respectively, allowing 1 bp mismatch and requiring minimum base quality of 20. Sequencing reads were aligned to the human genome using hg19 using bwa mem v0.7.15[23]. Bam files were sorted and indexed using samtools v1.3.1[24]. Reads with mapping quality <30 or unmapped, supplementary alignments, or not primary alignments were excluded from downstream analysis. Fragment size distribution and genomic coverage was calculated using Picard tools. One plasma sample was dropped from further analysis due to low coverage (<0.001x mean coverage). The modal fragment size and distance between fragment size peaks was calculated using a custom R script. Plasma and urine controls were pooled by merging reads using samtools.

Nucleosome coverage, fragment end position, and fragment end nucleotide frequencies

In a region with strongly positioned nucleosomes independent of tissue type[10], the physical coverage from pooled plasma and urine controls was compared. For ease of visualization, depth of coverage was min-maxed (normalized data from 0 to 1) and a rough local polynomial regression fitting (LOESS) regression with a span of 0.02 was applied. The mean smoothed physical coverage was calculated by centering all peaks in the region at their local maxima, estimated by inflection point.

To investigate the distance of fragment start and end sites in urine and plasma relative to their nearest nucleosome centers, a published plasma-based nucleosome occupancy map (CH01) was used as a reference[3]. Paired end reads were summarized as fragments with their 3' and 5' position into a bed file using BEDTools v2.26.0[25]. Further analysis was carried out in R using the GenomicRanges package. The bed file of all fragments was intersected with the nucleosome occupancy track. For each overlap hit, the distance of the fragment start and end position to the center of the respective nucleosome hit was calculated. In order to avoid fragments that might span more than one nucleosome, only 50-200 bp fragments were used for this analysis.

Using the fragments bed file, two additional bed files were created summarizing positions 10 bp upstream and downstream of fragment start and end site respectively. The genomic sequence of those regions was extracted using bedtools and calculated the mean per base mono-nucleotide frequencies using Homertools. The start and end sequence motifs were generated in R using the ggseqlogo package.

Nucleosome map generation

Published scripts based on window protection scores were used to create nucleosome occupancy maps using plasma and urine data[3]. For plasma samples, similar parameters as previously published were used: minimum fragment size of 120 bp, maximum fragment size of 180 bp, and window of 120 bp. For urine samples, the following parameters were used: minimum fragment size of 64 bp, maximum fragment size of 196 bp, and window of 120 bp. The parameters for urine cfDNA were changed to accommodate differences in fragment size distribution. To compare plasma and urine maps with previously published tracks, the fraction of nucleosome calls that overlapped with CH01, the peak-to-peak distance between adjacent peaks (interpeak distance), and the distance to the nearest peak of CH01 were calculated. The analysis was carried out in R using the GenomicRanges package.

cfDNA characteristics in open and closed chromatin regions

All autosomes in the hg19 human genome were tiled into 500 kb non-overlapping bins. Bins with mappability score <0.9, and bins within and or near the centromeric regions were executed, resulting in 4,975 bins. Each bin was annotated as compartment A (transcriptionally active and enriched for open chromatin) and compartment B (transcriptionally silent and enriched for closed chromatin) based on annotations from a previously published Hi-C chromatin contact map of lymphoblastoid cell lines (GM12878)[16]. The median interpeak distance in each bin was calculated for the plasma and urine nucleosome maps using the GenomicRanges package. Median fragment size in each bin was calculated using the Rsamtools package.

Inference of tissue of origin by comparison with DNase Hypersensitive Sites

To infer tissue of origin for plasma and urine cfDNA, median fragment size (MFS) was calculated in each of the 500 kb bins and median values were normalized to a z-score (subtracting MFS in each bin to the mean MFS in all bins and dividing by the standard deviation of MFS in all bins). Bins with negative z-scores represent regions with higher fraction of shorter fragments, and bins with positive z-scores represent regions with higher fraction of longer fragments. One hundred and sixteen (116) DHS call sets of different cell lines published earlier were processed in a similar manner[17]. The DHS data and annotations were downloaded from https://resources.altius.org/publications/Science _Maurano_Humbert_et_al/. For each call set, the number of DHS regions annotated in each 500 kb bin was calculated and normalized the counts to a z-score. Bins with negative z-scores represent regions with closed chromatin regions and bins with positive z-scores represent regions with open chromatin regions. The cosine similarity between the z-score vector for individual and pooled cfDNA samples and negative z-score vector for all DHS callsets were calculated. The cosine similarity between two vectors A and B can be calculated as: $\frac{A \cdot B}{\|A\| \|B\|}$. To evaluate individual plasma and urine samples, the cosine similarity was quantile normalized (R preprocessCore package) in order to maintain both, cell line ranking and continuous nature of the metric. The mean quantile normalized cosine similarity (MQNCS) was calculated for all bone marrow, lymphoid or myeloid cell lines (n=24) and renal cell lines (n=4) for individual cfDNA samples.

Inference of tissue of origin by comparison with gene expression

Using the previously generated cfDNA fragment bed files, 61-800 bp fragments were trimmed from both ends to contain 30 bp region downstream and upstream from the center (for odd fragment sizes we rounded the decimal down to closest integer). 20-60 bp fragments were left untrimmed. The trimmed fragment bed files were converted to bam files using bedtools. Trimmed fragment coverage was calculated around the transcription start sites (TSS) of all genes in hg19 autosomes using the Rsamtools package. The coverage in TSS $\pm$ 1000 bp window was normalized around the TSS of all genes by mean depth in TSS - 3000 bp to TSS - 1001 bp and TSS + 1001 bp to TSS + 3000 bp regions. The normalized coverage was further corrected by the coding strand direction. The strand corrected normalized coverage around the TSS $\pm$ 1000 bp window was averaged across genes with similar gene expression values in plasma, as published earlier[4]. To infer tissue of origin using TSS coverage, samples with mean genomic coverage >3x were included (pooled plasma, pooled urine, 10 individual plasma controls, and 28 individual urine controls). The raw read depth coverage was calculated -150 bp to +50 bp around the TSS (Nucleosome Depleted Region coverage) for all genes in hg19 autosomes and correlated to their respective expression values from 64 human cell lines and 37 primary tissues obtained from the Human Protein Atlas using Spearman's rank correlation coefficient (Spearman's rho). The change in rank was also assessed between pooled plasma and urine samples. To see whether this trend was consistent in individual plasma and urine samples, the Spearman's rho (R preprocessCore package) was quantile normalized for all 64 human cell lines and 37 primary tissues across all individual samples (10 plasma and 28 urine) in order to maintain both rank and the continuous nature of the metric. The mean quantile normalized Spearman's rho (MQNSR) was calculated for all bone marrow, lymphoid or myeloid tissues and cell lines (n=16) and two renal cell lines (RPTEC-TERT1 and RT4) for individual samples.

Aberrant fragmentation ends and fragment end nucleotide frequency in cancer patients

Reads from 20 healthy urine samples (12 females and 8 males) were pooled using samtools and a urine reference nucleosome map (URNP) was built using parameters described earlier. Individual fragment bed files were intersected with URNP using GenomicRanges package in R. For each overlap hit, the distance of the fragment start and end position to the center of the respective nucleosome hit was calculated. The fraction of fragments that started or ended within 60 bp downstream or upstream of nucleosome center was calculated. These were counted as aberrant fragments, as they are being cleaved within or close to the nucleosome centers observed in reference samples. The fraction of aberrant fragments in 20 control urine samples used to generate the URNP was compared with urine samples from another 10 controls, 10 pediatric cancer patients, and 12 pancreatic cancer patients. The predictive performance of fraction of fragments with aberrant ends to distinguish between healthy and cancer samples was calculated using receiver operator curve (ROC) analysis (pROC R package). Since the fraction of fragments with aberrant ends in training and test control samples was similar, all 30 controls samples were used in the ROC analysis. ROC analyses were conducted on pediatric and pancreatic cancer samples separately and in combination.

Using the urine cfDNA fragment bed files from controls and cancer patients, two additional bed files were created summarizing positions 10 bp upstream and downstream of fragment start and end site respectively. The genomic sequence of those regions was extracted using bedtools and calculated the mean per base mono- and di-nucleotide frequencies as well as cumulative frequency of CpG, total G+C, total A+G, and total A+C using Homertools. For each individual sample, the various per base frequencies at fragment start and end sites were summarized in a single vector of length 168 in R. The nucleotide frequency vector from all urine samples was concatenated into one matrix (52 x 168). To reduce dimensions, Multidimensional Scaling (MDS) was carried out to reduce the data to 4 dimensions (52 x 4). It was then whether nucleotide frequencies at fragment start and end sites could classify between healthy samples from cancer samples by plotting various combinations of the 4 dimensions. Nucleotide frequencies at fragment start and end sites are referred to as AFEM (aberrant fragment end motif). To calculate the predictive performance of AFEM to distinguish between healthy and cancer samples, logistic regression was fitted using base *glm* function in R to the 4 MDS dimensions and the predictive probability from the model was used to conduct ROC analysis. ROC analyses were conducted on pediatric and pancreatic cancer samples separately and in combination. The 4 MDS dimensions and AFE were also combined, and an integrated ROC analysis was conducted using similar steps.

Aberrant fragmentation ends in copy number aberration regions

To investigate whether the AFE was affected by underlying copy number changes in the tumor, data generated using exome sequencing of tumor and germline DNA samples from 2 patients with pediatric cancers and 4 patients with pancreatic cancer was used. Regions with copy number aberrations were calculated using the R package Sequenza[26]. For each of the 6 patients, the 4975 bins were marked as copy number neutral, loss, or gain. Any bins that were partially segmented into two different copy number states were removed. The AFE in each of the filtered 500 kb bins was calculated for urine samples from the 6 cancer patients and the 10 controls not used to build the URNP. For each patient, the AFE ratio in bin i was calculated as the AFE in bin *i* of patient sample divided by the mean AFE in bin *i* of the 10 healthy urine samples, as shown in Equation 1.

$$AFE\ ratio\ _{patient,i} = \frac{AFE_{paitent,i}}{mean(AFE_{normals,i}})) \qquad (1)$$

Background distribution of AFE ratio for each bin was also calculated using the 10 control urine samples, by picking one sample and calculating its AFE ratio using the mean AFE of the remaining 9 samples. An example for one healthy sample is shown in Equation 2. This was repeated for all 10 controls.

$$AFE\ ratio\ _{normal\ 1,i} = \frac{AFE_{normal\ 1,i}}{mean(AFE_{normal\ 2-10,i}}) \qquad (2)$$

The z-score of AFE ratio in bin *i* was then calculated as the AFE ratio in bin *i* of a patient sample subtracted by the mean and divided by the standard deviation of background AFE ratio in bin i of the 10 healthy samples, as shown in Equation 3.

$$Z-score\ _{patient,i} = \frac{AFE\ ratio\ _{paitent,i} - mean(AFE\ ratio\ _{normals,i})}{sd(AFE\ ratio\ _{normals,i})}) \qquad (3)$$

For each patient the distribution of AFE ratio z-scores in copy number neural, loss, and gain bins was compared.

Urine Histone Analysis by Mass Spectrometry

As urine proteins are especially subject to hydrolysis due to urea, proteins encapsulated in extracellular vesicles (EVs) were isolated from urine to increase protein coverage. EVs were isolated from 10 ml pooled commercially available normal human urine (Lee Biosolutions, Maryland Heights, MO) using the ExoEasy Maxi kit (Qiagen, Germantown, MD) and following the manufacturer's instructions. 4 mL of the flow-through fraction was processed by trichloroacetic acid (TCA) precipitation in 4:1 urine:acid ratio. Briefly, 1 ml of pre-chilled 100% TCA was added to 4 ml of urine flow-through, vortexed, and chilled for 1 h on ice. The sample was then centrifuged at 11,000 RCF for 30 min. After discarding the supernatant, pellets were first covered with 0.1% HCl in 100% ice cold acetone, then centrifuged at 11,000 RCF for 2 min. This step was repeated once again with 100% ice cold acetone. Pellets were then dried using nitrogen air flow and re-suspended in 200 $\mu$l of 50 mM ammonium bicarbonate for bicinchoninic acid (BCA) quantification. Equimolar amounts of the captured EV fraction and the TCA precipitated flow-through fraction were then diluted 2x with solution containing 50 mM Tris- HCl pH 7.0, 1X HALT (Thermo Fisher Scientific, San Jose, CA) and lysed by sonication on a UTR200 cup sonicator (Hielscher Ultrasound Technology, Teltow, Germany). Lysed fractions were incubated with TCEP (Thermo Fisher Scientific, Waltham, MA) at a final concentration of 5 mM for 45 min at 60 °C on thermoshaker, 450 rpm, followed by incubation with iodoacetemide (Sigma-Aldrich, Saint Louis, MO) to final concentration of 10 mM for 30 min at room temperature in the dark. Each fraction was then diluted threefold with 50 mM Tris-HCl. Polypeptides were trypsin digested at a ratio of 1:50 (Promega), overnight at 37 °C, and subjected to solid phase extraction. Peptides in solution were dried by speed vacuum and

reconstituted in 50 mM NH4OH and quantified by BCA (Thermo Fisher Scientific). Basic reverse phase fractionation was carried out on 8 $\mu$g of tryptic peptides using an XBridge BEH C18 column (130 Å, 3.5 $\mu$m particle size, 4.6 mm x 100 mm) (Waters, Milford, MA) connected to a U3000 UHPLC (Thermo Fisher Scientific) system operating at 0.3 ml/min flow-rate. Peptides were fraction-collected into a 96-deep well plate using a gradient of acetonitrile and water, and 10% aqueous 50 mM Ammonium Hydroxide (pH 10)34. The resulting 96 fractions were concatenated into 6 analytical fractions, vacuum-dried and reconstituted in 6 $\mu$l of aqueous 0.1% formic acid solution for LC-MS/MS analysis.

Mass spectrometry acquisition was performed in top-speed data-dependent mode (3 second duty cycle) on an Orbitrap Fusion Lumos Tribrid (Thermo Fisher Scientific) mass spectrometer coupled to a nanoAcquity UPLC system (Waters). Peptides were separated on a PepMap RSLC C18 EasySpray C18 column (100 Å, 2 $\mu$m particle size, 75 $\mu$m x 25 cm) kept at 50 °C with a 120 min gradient from 3% to 30% to 90% acetonitrile in 0.1% formic acid, at a flow-rate of 350 nl/min. The mass spectrometer was operated with the following parameters: ion transfer tube temperature of 275 °C, spray voltage of 2400 V, MS1 in Orbitrap with a resolution of 120K and mass range of 400-1500 m/z, most abundant precursors (excluding undetermined and +1 charge state species) were selected for MS2 measurement in the iontrap following HCD fragmentation with 35% collision energy; dynamic exclusion was set to 60 s. Mass spectra were searched using Proteome Discoverer (v2.1.0.388, Thermo Fisher Scientific) and Mascot (Matrix Science, Boston, MA) on a human UniprotKB (Swissprot, June 2017) database allowing for two missed cleavages, fixed cysteine carbamidomethylation and variable methionine oxidation, a 10 ppm precursor and 0.6 Da fragment mass tolerance. Percolator was employed with a target-decoy strategy to determine false discovery rates at peptide and protein level

Evaluation of preanalytical variability in urine cfDNA samples

Five healthy adults were enrolled at the Translational Genomics Research Institute, Phoenix, AZ, under IRB protocol number 20142638 approved by Western IRB. Informed consent was obtained from all participants. First void of the day urine sample was self-collected off-site in a sterile cup containing 0.5M EDTA for a minimum concentration of 10 mM. Urine from a subsequent void was collected on-site in a sterile cup without any additive. To process these samples, 10 mL aliquots were made from both samples. For the subsequent void sample, 0.2 mL of 0.5M EDTA was added to an aliquot immediately. The remaining aliquots were stored at room temperature for 30, 60, 120, and 240 minutes prior to addition of EDTA and further processing. Aliquots were centrifuged at 1600g for 10 minutes at 4 °C and the supernatant was stored at -80 °C until extraction. cfDNA was extracted from 10 mL of urine with the MagMAX Cell-Free DNA Isolation Kit (ThermoFisher Scientific) according to manufacturer instructions and eluted in 25 $\mu$l. Total DNA was quantified with the Qubit dsDNA HS Assay Kit (ThermoFisher Scientific). Whole genome sequencing libraries were generated with 0.5 - 6.0 ng input according to manufacturer instructions using the SMARTer ThruPLEX Plasma-Seq kit (Takara). Sequencing was performed on Illumina NextSeq to generate 75 bp paired-end reads. Analysis of fragment size was performed as described above.

**Example 2. Development of cfDNA Detection in Urine and Plasma and Evaluation of Assay Performance in Reference Samples.**

To investigate fragment size distribution with high resolution and compare with plasma samples, whole genome sequencing (WGS) of 30 urine and 15 plasma cfDNA samples collected from unrelated healthy volunteers (mean physical coverage of 7x and total coverage 196x in urine, mean 3.6x and total coverage 58x in plasma) was performed. In plasma cfDNA, a modal fragment size of 167 bp was measured, as reported previously[8] **(FIG. 1A)**. In urine cfDNA, the modal fragment size in 23/30 samples was 80-81 bp. In an additional 6/30 samples, the modal fragment size was 111-112 bp **(FIG. 1B)**. In both urine and plasma, a 10 bp step pattern was identified but the amplitude of each fragment size peak was much greater in urine **(FIG. 1C)**. While the size distribution of plasma cfDNA showed one predominant peak at 167 bp, fragment sizes in urine cfDNA showed multiple sharp peaks between 40 bp and 120 bp that were more evenly distributed relative to the 81 bp mode.

The modal size of 80-81 bp in the majority of urine samples suggests that the cfDNA may be associated with a stable intermediate product of histone-DNA interaction and nucleosome assembly. In vitro studies show that a histone $H3_2H4_2$ tetramer is the most energetically favorable intermediate component during stepwise nucleosome assembly. The intermediate tetrasome binds the central ~67 bp region of the DNA originally wrapped within mono-nucleosomes[9]. To evaluate whether a similar mechanism may explain fragment sizes in urine cfDNA, physical sequencing coverage was compared between plasma and urine in a genomic region on chromosome 12 that has been previously reported to have consistent strongly positioned nucleosomes[10]. In plasma and urine cfDNA, there were periodic peaks in coverage that were consistent in positioning across the two sample types **(FIG. 2A)**. Individual urine peaks were narrower and occupied the center of corresponding plasma peaks **(FIG. 2B)**. The average distance between consecutive peaks was 188 bp and 187 bp for plasma and urine samples, respectively. To investigate whether coverage of urine cfDNA fragments was consistent with nucleosome positioning across the genome, four independent nucleosome maps were generated using a single pool of plasma samples from healthy individuals (HP) and three independent pools of urine samples (healthy individuals HU, and two sets of cancer patients CU1 and CU2). There were 11.8 million, 7.2 million, 7.9 million and 6.7 million nucleosome peaks identified, respectively. The HP were evaluated against another well-characterized and published plasma-based nucleosome positioning map (CH01). 70% of nucleosome positions called in HP overlapped with CH01, while 64% of nucleosome positions called in CH01 overlapped with HP.

When the 3 independent urine maps were compared with each other, 49%-60% peaks overlapped in pairwise comparisons. The overlap between plasma and urine, however, was much lower. 30%-38% of nucleosome peaks in plasma map CH01 overlapped with any of the urine maps while the converse overlap was 58%-62% **(FIG. 2C)**. Non-overlapping peaks had significantly lower confidence scores compared to overlapping peaks. The modal distance between consecutive adjacent nucleosome peaks (periodicity) across plasma and urine maps was similar and consistent with periodic nucleosome positioning (177 bp to 184 bp respectively; **FIG. 2D)**. When any two nucleosome positioning maps were compared (plasma-plasma, urine-urine, or plasma-urine comparisons), the distance between the centers of the corresponding nearest peaks was predominately zero. The spread of nearest peak distances, however, was narrowest for plasma-plasma comparisons, wider for urine-urine comparisons, and widest for plasma-urine comparisons **(FIG. 2E)**. These differences between urine and plasma-based maps suggest a combination of two sources of variation. In urine-urine comparisons, lower overlap in nucleosome peaks and greater spread of distances between nearest peaks are likely driven by fewer and less precise nucleosome positions inferred from urine due to greater fragmentation of cfDNA in urine compared to plasma. In urine-plasma comparisons, the additional discordance in peak overlap and even greater spread of distances between nearest peaks observed in urine-plasma comparisons may in part be the result of differences in nucleosome positions in cell types predominately contributing cfDNA in plasma and urine.

To further assess if urine cfDNA fragments are informative of genome-wide nucleosome position in contributing cells, the interpeak distances between adjacent nucleosomes was compared to cfDNA fragment sizes within open and closed chromatin regions. Open and closed chromatin regions were identified in a published dataset generated using Hi-C chromatin contact analysis of a lymphoblastoid cell line (GM12878). This dataset annotated the genome into two compartments: compartment A is enriched for transcriptionally active open chromatin regions and compartment B is enriched for transcriptionally silent closed chromatin regions. The median interpeak distances and mean fragment sizes were calculated across the genome in non-overlapping windows of 500 kb. The plasma-based nucleosome positioning map (HP) showed shorter distances between adjacent nucleosomes on average in open chromatin regions compared to closed chromatin regions (median interpeak distances of 190 bp and 193 bp, respectively, $p<2\times10^{-16}$, Student's t-test; **FIG. 2F)**. A similar trend was observed for interpeak distances in the urine-based map (HU, median interpeak distances of 301 bp and 302 bp, respectively, p=0.049, Student's t-test; **FIG. 2G)**. In addition, we found that on average across the genome, cfDNA fragments are more degraded and shorter in size in open chromatin regions compared to closed chromatin regions in both, plasma (mean fragment size of 169 bp and 170 bp, respectively, $p<2\times10^{-16}$, Student's t-test; **FIG. 2H)** and urine samples (mean fragment size of 132 bp and 133 bp, respectively, $p<2\times10^{-16}$, Student's t-test; **FIG. 2I)**. The difference in fragment size between open and closed chromatin regions was also robust to changes in the window size used for these calculations, whether when it is decreased to 50 kb or increased to 1000 kb.

To explore whether cfDNA fragment sizes are associated with local differences in chromatin accessibility, window-level correlation between cfDNA fragment size, and open/closed chromatin annotation was evaluated. For each individual sample, the median fragment size within non-overlapping 500 kb windows was calculated for all autosomes and all median values were normalized to z-scores. These scores were compared with the Hi-C annotated data from the lymphoblastoid cell line GM12878. Consistent with recent results, genomic windows with negative fragment size z-scores (shorter fragments than median fragment size) were associated with open chromatin regions and windows with positive fragment size z-scores (longer fragments than median fragment size) were associated with closed chromatin regions **(FIGs 3A-3C)**. This association was stronger for plasma samples than urine samples (cosine similarity of 0.53 and 0.37 respectively), consistent with lymphoblastoid origin of the tested cell line and a higher contribution of lymphoid cells into plasma cfDNA. To evaluate whether a different cell type may show stronger association with urine cfDNA, this analysis was expanded to multiple cell types using a published dataset describing DNase I hypersensitive sites (DHS) across 116 cell lines and tissues. For each cell line or tissue, the number of DHS regions annotated in non-overlapping 500 kb windows was calculated for all autosomes and all counts were normalized to z-scores, transformed such that open chromatin regions with greater DHS sites will have a negative z-score and vice versa. Using these two sets of z-scores for fragment size and DHS sites across all windows, the cosine similarity between the fragment size vector from each individual sample and the DHS vector was calculated for each cell line or tissue. In open chromatin regions, a greater number of DHS sites and shorter cfDNA fragments are anticipated and therefore, the cosine similarity is expected to be positive. For pooled plasma cfDNA, the highest cosine similarity was observed with lymphoid or myeloid cells. In contrast, for pooled urine cfDNA, the highest cosine similarity was observed with epithelial, renal epithelial and renal cortical cells **(FIGs 3D-3E).** The mean quantile normalized cosine similarity (MQNCS) for lymphoid or myeloid cells (n=21) was higher in plasma samples compared to urine samples (p<0.001, Student's t-test). Conversely, MQNCS for renal cells (n=4) was lower in plasma samples compared to urine samples (p<0.01, Student's t-test; **FIGs 3F-3G).** These results suggest renal and uro-epithelial cells contribute a higher fraction of cfDNA in urine, compared to plasma. In urine samples, cell-type specific MQNCS scores were much more variable compared to plasma samples, suggesting that tissue-specific contributions in urine samples may be more variable.

To evaluate tissue contributions in urine using an alternative approach, cfDNA coverage was analyzed around transcription start sites in urine and plasma. A recent study reported depletion of coverage in plasma cfDNA at the nucleosome-depleted region (NDR), from ~150 bp upstream to ~50 bp downstream of transcription start sites (TSS) and an association between NDR depletion and higher gene expression. A similar pattern was observed when comparing cfDNA coverage at the NDR region in plasma and urine samples with gene expression in plasma. Plasma and urine cfDNA, however, were discordant in terms of overall cfDNA coverage in the 2 kb region around TSS. In plasma cfDNA, the earlier reported relative depletion in overall coverage in the highest expressed genes was confirmed. In urine cfDNA, however, coverage in the 2 kb region around the TSS was higher than surrounding loci and coverage was higher downstream of TSS than upstream, particularly for highly expressed genes **(FIGs 4A-4B).** This difference in profile of relative sequencing coverage in the TSS region between urine and plasma cfDNA was unrelated to cell type used to measure gene expression. The coverage profile observed in urine cfDNA is similar to prior analyses of nucleosome occupancy around TSS based on DNA sequence content, suggesting that it may be driven by physiological conditions driving nucleosome unfolding and digestion in urine. Since an association between coverage depletion at NDR and gene expression was seen in both plasma and urine cfDNA samples, genome-wide correlation between NDR sequencing coverage and gene expression was evaluated in 101 human cell lines and primary tissues in the Human Protein Atlas to infer tissue of origin. For each plasma or urine sample, coverage was measured at the NDR, from -150 bp to +50 bp around TSS of protein coding genes on autosomes. Spearman's rank correlation coefficient (Spearman's rho) was calculated between mean NDR coverage and individual gene expression values in each cell line or tissue. A stronger negative correlation was expected for cell types contributing greater amounts of cfDNA in each sample type. For plasma samples, the three most negative correlations were observed with lymphoblast cell lines. In contrast, for the urine samples, the three most negatively correlated cell lines included two epithelial cell lines (kidney and endometrial) and a lymphoblast cell line. To highlight the key differences between the two sample types, the change in cell line and tissue ranks was evaluated between plasma and urine analyses. The two largest decreases in ranks by 27 positions were observed for a cell line of monocyte origin (THP-1) and for bone marrow tissue, consistent with lower contribution of these in urine compared to plasma. The largest increases in ranks by 33 positions were observed for two cell lines of urinary bladder (RT4) and renal cortical origin (RPTEC; **FIG. 4C),** consistent with higher contribution of these in urine compared to plasma. RPTEC also ranked 6/116 in the cosine similarity analysis of fragment size and DHS sites for urine cfDNA described earlier (compared to a rank of 61/116 for plasma cfDNA). The mean quantile normalized Spearman's rho (MQNSR) for the cell line of monocyte origin (THP-1) was lower in plasma compared to urine samples (p<0.001, Student's t-test; **FIG. 4D).** Conversely, MQNSR for renal epithelial cell line (RPTEC) and urinary bladder cell line (RT4) was lower in urine compared to plasma samples (both p<0.001, Student's t-test; **FIG. 4E-4F).**

If nucleosome positioning maps inferred from cfDNA are representative of how most fragments are protected from degradation in plasma or urine, a higher density of fragment ends is expected at the periphery of corresponding nucleosome dyads compared to the center. This trend was observed for both plasma and urine cfDNA samples. When compared with a plasma nucleosome positioning map (CH01), the distribution of fragment start and end site distances in plasma cfDNA samples showed distinct modes at 77 bp upstream and downstream of nucleosomal dyad peaks and reduced representation within the nucleosome core. When urine cfDNA samples were compared to a urine nucleosome positioning map (HU), the distribution of fragment start and end sites showed modes at 67 bp on either side of the dyad. Frequency of fragment ends was lower within the nucleosome core, although this was less pronounced in urine cfDNA compared to plasma. A change in slope of the distribution of urine cfDNA fragment ends was observed at 40 bp on either side of the dyad **(FIG. 5A).** These observations suggest in plasma, most cfDNA fragment ends are observed at expected genomic loci that can be inferred using plasma-based nucleosome positioning maps. This is also true for a majority of urine cfDNA fragment ends but some are still observed within nucleosome core regions that one would expect to be protected, relative to a urine-based nucleosome positioning map. This is likely due to on-going degradation and only transient protection of urine cfDNA by association with histones, and a higher fraction of sub-nucleosomal shorter fragment sizes observed in urine cfDNA.

Nucleotide frequencies observed at cfDNA fragment ends were also compared between urine and plasma. As reported recently, there was a consistent pattern of nucleotide frequencies across 10 bp regions upstream and down-stream of fragment start and end sites in plasma cfDNA, conserved across plasma samples. In urine cfDNA, there was a different pattern from plasma which was also conserved across urine samples **(FIG. 5B-5C).** To evaluate whether these sequence preferences vary for different fragment lengths, fragments were divided into bins by fragment size (with 10 bp increments such as 65 bp to 74 bp, 75 bp to 84 bp, and so on)21. In both plasma and urine, these patterns were conserved regardless of fragment size. These observations indicate that there may be different enzymes responsible for DNA degradation in plasma and urine. A recent study suggests that DNase1-like3 is the predominant enzyme degrading chromatin in plasma. In contrast, DNase1, an enzyme highly active in urine, has preferential activity for naked DNA after DNA-bound proteins are removed. Indeed, another recent paper showed that DNA fragment ends resulting from DNase1-like3 digestion show strong preference for cytosine (C) as the first base (as observed in plasma cfDNA). In contrast, DNA fragment ends resulting from DNase1 digestion showed strong preference for thymine (T) as the first base (as observed in urine cfDNA).

**Example 3. Detection of Cancer-Specific cfDNA Fragments in Patient-Collected Urine Samples**

The findings thus far suggested that genome-wide distribution of urine cfDNA fragments are associated with chromatin accessibility in the contributing cell types and that relative to a urine-based nucleosome positioning map, a majority of urine cfDNA fragment ends are observed outside of the nucleosome core region. Because cancer cells are likely to have differences in nucleosome positioning compared to cells and tissues that routinely contribute cfDNA into urine in healthy individuals, cancer-derived cfDNA fragments in urine may deviate from expectations set by inferred nucleosome positioning maps at a higher rate **(FIG. 6A)**. Whole genome sequencing (WGS) was performed on pre-treatment urine samples from 10 patients with nonmetastatic pediatric solid cancers (mean physical coverage of 21.6x) and 12 patients with pancreatic adenocarcinoma including 7 patients with stage I-II and 5 patients with stage IV disease (mean physical coverage of 0.72x). Urine cfDNA fragment size distribution was consistent with that observed in healthy individuals. First, the fraction of aberrant fragments (with fragment ends within 65 bp of the nucleosome center) was measured in healthy individuals. To do so, a reference nucleosome positioning map was built using pooled data from 20/30 urine samples from healthy individuals (the training set, mean physical coverage of 155x). Against this map, the fraction of aberrant fragments was calculated in the training set and an additional 10 control samples (the test set). The mean fraction of aberrant fragments observed in the training and test sets was 35.0% and 35.4%, respectively (p>0.05, Student's t-test). Some background fraction of aberrant fragments was expected even in control urine samples **(FIG. 5A),** due to a higher abundance of shorter fragments in urine cfDNA samples and variability in contributing tissue types across healthy individuals. No significant difference in fraction of aberrant fragments was observed between the training and test sets, however, indicating that the reference map was robust to variations between healthy individuals. In contrast and consistent with the hypothesis, there was a significantly higher fraction of aberrant fragments in both sets of cancer patients compared to the training set (mean fraction of aberrant fragments was 36.6%, p<0.01, Student's t-test; **FIG. 6B).** To evaluate whether differences in chromatin accessibility and associated fragmentation sites in cancer cells also result in minor deviations in nucleotide frequencies at fragment ends, the mononucleotide frequencies were analyzed in the 10 bp region upstream and downstream of fragment start and end sites. There were no obvious differences between urine samples from cancer patients and controls in patterns of nucleotide frequencies seen at fragment ends. Multidimensional scaling, however, showed separation between healthy individuals and pancreatic cancer patients in the third dimension **(FIG. 6C).** Using thresholds for fraction of aberrant ends and for multiple dimensions of nucleotide frequency at fragment ends, the ability to distinguish urine samples from cancer patients was evaluated. Using either feature individually or a combination of the two, we were able to distinguish cancer patients from healthy individuals, achieving an area under the receiver operating characteristic curve of 85.3%-88.8% **(FIG. 6D).** These findings highlight the potential for genome-wide analysis of urine cfDNA fragmentation and positioning in improving cancer diagnostics, particularly for early detection of cancer.

To evaluate whether the increase in fraction of aberrant fragments was specifically driven by tumor DNA or associated with physiological differences in cancer patients, the fraction of aberrant fragments was compared between genomic regions that were gained, lost or neutral due to somatic copy number changes in the tumor. If the tumor tissue was at least a partial source of aberrant cfDNA fragments, the fraction of aberrant fragments was expected to be higher in copy number gain regions because the amount of tumor DNA contributed into urine by those regions will be greater. In 4/6 patients, the fraction of aberrant fragments was higher for genomic regions with copy number gains in tumor, compared to neutral and/or lost regions (one tailed p<0.05, Student's t-test; **FIG. 7).** In a fifth patient, no significant difference was observed (one-tailed p=0.079, Student's t-test). In one patient, a reverse trend was observed but the tumor genome of this patient had widespread copy number changes with no clear baseline copy number neutral region. In all 6 cases, urine cfDNA samples showed no copy number aberrations and no detectable tumor fraction was observed using a published method for copy number analysis.

**Example 4. Pre-analytical variation is observed in urine cfDNA fragmentation patterns.**

To evaluate the extent to which our results may be affected by pre-analytical variation such as time of voiding or sample processing artefacts, the cfDNA yield and fragmentation profile was compared across 30 urine samples obtained from 5 healthy individuals (3 male and 2 female). Each individual provided a first void of the day sample that they collected at home in a urine cup with EDTA additive (first void or FV sample). Self-reported time since last void for the FV sample was 4.5 to 8.6 hours (mean 7.1 hours). No further processing was performed until the sample arrived in the research lab (a delay of 2.6 to 5.7 hours, mean 3.7 hours). Each individual also provided a second sample on site in a urine cup without any additive and this was divided in 5 aliquots. Self-reported time since last void for the second sample was 1.3 to 6.0 hours (mean 4.2 hours). EDTA was added as soon as possible after collection to the first aliquot within 13 to 23 mins (mean 16 mins, T0 sample) and in the remaining aliquots at 30 mins (T30), 60 mins (T60), 120 mins (T120) and 240 mins thereafter (T240; **FIG. 8A**). There was no significant difference in total cfDNA yield between the first void and subsequent sample processed at T0 (p=0.53, Student's t test; **Fig. 8B**). All individuals showed a stable yield between T0 and T30 but 4/5 individuals showed an elevation at T60 that was sustained in later time points **(FIG. 8C)**. The fragment size distributions obtained from the two independent urine samples (FV and T0) were indistinguishable **(FIG. 8D).** Similarly, fragment size distributions obtained from multiple processing time points of the second urine sample were completely overlapping **(FIG. 8E).**

## Example 5. Detection of Cancer-Specific Aberrant Fragment Ends (AFEs) in Patient-Collected Plasma Samples

These aberrant fragment ends (AFEs) were quantified in plasma samples from cancer patients with metastatic melanoma and found highly significant elevations in the AFE fraction compared to healthy controls **(FIG. 9).** Moreover, AFE fraction was well correlated with tumor fraction in ctDNA (as measured using sWGS copy number analysis, the existing approach; $R=0.814$, $p=9.74 \times 10^{-11}$, Spearman's Correlation; **FIG. 10).** Even for samples with ctDNA levels at or near the limit of detection of the current approach (~3%), AFE fractions were significantly higher in plasma samples from cancer patients than in plasma samples from healthy volunteers in many cases.

Based on the data so far, a cfDNA-based nucleosome positioning map specific to different subtypes of cancer patients is able to be inferred from plasma DNA using WGS. Comparing such a map to healthy control nucleosome maps will allow quantification of cfDNA fragments specifically contributed by breast cancer or melanoma cells (for example but not limited to these cancer types), enabling an even higher resolution for ctDNA detection and quantification.

## REFERENCES

1 Murtaza, M. & Caldas, C. Nucleosome mapping in plasma DNA predicts cancer gene expression. Nat Genet 48, 1105-1106, doi:10.1038/ng.3686 (2016).

2 Cristiano, S. et al. Genome-wide cell-free DNA fragmentation in patients with cancer. Nature, doi:10.1038/s41586-019-1272-6 (2019).

3 Snyder, M. W., Kircher, M., Hill, A. J., Daza, R. M. & Shendure, J. Cell-free DNA Comprises an In Vivo Nucleosome Footprint that Informs Its Tissues-Of-Origin. Cell 164, 57-68, doi:10.1016/j.cell.2015.11.050 (2016).

4 Ulz, P. et al. Inferring expressed genes by whole-genome sequencing of plasma DNA. Nat Genet 48, 1273-1278, doi:10.1038/ng.3648 (2016).

5 Mouliere, F. et al. Enhanced detection of circulating tumor DNA by fragment size analysis. Sci Transl Med 10, doi:10.1126/scitranslmed.aat4921 (2018).

6 Cheng, T. H. T. et al. Genomewide bisulfite sequencing reveals the origin and time-dependent fragmentation of urinary cfDNA. Clin Biochem 50, 496-501, doi:10.1016/j.clinbiochem.2017.02.017 (2017).

7 Tsui, N. B. et al. High resolution size analysis of fetal DNA in the urine of pregnant women by paired-end massively parallel sequencing. PLoS One 7, e48319, doi:10.1371/journal.pone.0048319 (2012).

8 Lo, Y. M. et al. Maternal plasma DNA sequencing reveals the genome-wide genetic and mutational profile of the fetus. Sci Transl Med 2, 61ra91, doi:10.1126/scitranslmed.3001720 (2010).

9 Thastrom, A., Bingham, L. M. & Widom, J. Nucleosomal locations of dominant DNA sequence motifs for histone-DNA interactions and nucleosome positioning. J Mol Biol 338, 695-709, doi:10.1016/j.jmb.2004.03.032 (2004).

10 Gaffney, D. J. et al. Controls of nucleosome positioning in the human genome. PLoS Genet 8, e1003036, doi1:10.1371/journal.pgen.1003036 (2012).

11 Chandrananda, D., Thorne, N. P. & Bahlo, M. High-resolution characterization of sequence signatures due to non-random cleavage of cell-free DNA. BMC Med Genomics 8, 29, doi:10.1186/s12920-015-0107-z (2015).

12 Chen, S. et al. A Study of Cell-free DNA Fragmentation Pattern and Its Application in DNA Sample Type Classification. IEEE/ACM Trans Comput Biol Bioinform, doi:10.1109/TCBB.2017.2723388 (2017).

13 Serpas, L. et al. Dnase1l3 deletion causes aberrations in length and end-motif frequencies in plasma DNA. Proc Natl Acad Sci U S A 116, 641-649, doi:10.1073/pnas.1815031116 (2019).

14 Nadano, D., Yasuda, T. & Kishi, K. Measurement of deoxyribonuclease I activity in human tissues and body fluids by a single radial enzyme-diffusion method. Clin Chem 39, 448-452 (1993).

15 Napirei, M., Ludwig, S., Mezrhab, J., Klockl, T. & Mannherz, H. G. Murine serum nucleases--contrasting effects of plasmin and heparin on the activities of DNase1 and DNase1-like 3 (DNase1l3). FEBS J 276, 1059-1073, doi:10.1111/j.1742-4658.2008.06849.x (2009).

16 Baldi, S., Krebs, S., Blum, H. & Becker, P. B. Genome-wide measurement of local nucleosome array regularity and spacing by nanopore sequencing. Nat Struct Mol Biol 25, 894-901, doi:10.1038/s41594-018-0110-0 (2018).

17 Maurano, M. T. et al. Systematic localization of common disease-associated variation in regulatory DNA. Science 337, 1190-1195, doi:10.1126/science.1222794 (2012).

18 Sun, K. et al. Orientation-aware plasma cell-free DNA fragmentation analysis in open chromatin regions informs tissue of origin. Genome Res 29, 418-427, doi:10.1101/gr.242719.118 (2019).

19 Liu, Y. et al. Spatial co-fragmentation pattern of cell-free DNA recapitulates in vivo chromatin organization and identifies tissues-of-origin. bioRxiv, 564773, doi:10.1101/564773 (2019).

20 Lu, T. & Li, J. Clinical applications of urinary cell-free DNA in cancer: current insights and promising future. Am J Cancer Res 7, 2318-2332 (2017).

21 Fujii, T. et al. Mutation-Enrichment Next-Generation Sequencing for Quantitative Detection of KRAS Mutations in Urine Cell-Free DNA from Patients with Advanced Cancers. Clin Cancer Res 23, 3657-3666, doi:10.1158/1078-0432.CCR-16-2592 (2017).

22 Markus, H. et al. Evaluation of pre-analytical factors affecting plasma DNA analysis. Sci Rep 8, 7375, doi:10.1038/s41598-018-25810-0 (2018).

23 Li, H. Aligning sequence reads, clone sequences and assembly contigs with BWA-MEM. arXiv preprint arXiv:1303.3997 (2013).

24 Li, H. et al. The Sequence Alignment/Map format and SAMtools. Bioinformatics 25, 2078-2079, doi:10.1093/bioinformatics/btp352 (2009).

25 Quinlan, A. R. & Hall, I. M. BEDTools: a flexible suite of utilities for comparing genomic features. Bioinformatics 26, 841-842, doi:10.1093/bioinformatics/btq033 (2010).

26 Favero, F. et al. Sequenza: allele-specific copy number and mutation profiles from tumor sequencing data. Ann Oncol 26, 64-70, doi:10.1093/annonc/mdu479 (2015).

27 Batth, T. S., Francavilla, C. & Olsen, J. V. Off-line high-pH reversed-phase fractionation for in-depth phosphoproteomics. J Proteome Res 13, 6176-6186, doi: 10.1021/pr500893m (2014).

28 The, M., MacCoss, M. J., Noble, W. S. & Kall, L. Fast and Accurate Protein False Discovery Rates on Large-Scale Proteomics Data Sets with Percolator 3.0. J Am Soc Mass Spectrom 27, 1719-1727, doi:10.1007/s13361-016-1460-7 (2016).

## Claims

1. A method of detecting an aberrant end cell free DNA (cfDNA) fragmentation profile of a subject, the method comprising the steps of:

   providing a sample obtained from the subject;
   extracting cfDNA from the sample to obtain cfDNA fragments;
   performing whole genome sequencing on the cfDNA fragments extracted from the sample to generate sequencing reads for the cfDNA fragments; and
   determining from the sequencing reads a distribution of start and end sites of the cfDNA fragments;
   determining a nucleotide frequency at start sites and end sites of the cfDNA fragments relative to a nucleotide frequency at start sites and end sites of a control sample, thereby identifying aberrant fraction ends in the sample from the subject; and
   determining an aberrant ends fraction of the cfDNA fragments from the sample to generate the aberrant end cfDNA fragmentation profile of the subject.

2. A method of detecting disease or an abnormal cell type in a subject using an aberrant end cfDNA fragmentation profile of a subject, the method comprising the steps of:

   providing a sample obtained from the subject;
   extracting cfDNA from the sample to obtain cfDNA fragments;
   performing whole genome sequencing on the cfDNA fragments extracted from the sample to generate sequencing reads for the cfDNA fragments;
   determining from the sequencing reads a distribution of start and end sites of the cfDNA fragments;

determining a nucleotide frequency at start sites and end sites of the cfDNA fragments relative to a nucleotide frequency at start sites and end sites of a control sample, thereby identifying aberrant fraction ends in the sample from the subject;

determining an aberrant ends fraction of the cfDNA fragments from the sample to generate the aberrant end cfDNA fragmentation profile of the subject;

comparing the subject's aberrant end cfDNA fragmentation profile to an aberrant end cfDNA fragmentation profile from a control sample; and

detecting the presence of disease or an abnormal cell type in the subject based on the subject's aberrant end cfDNA fragmentation profile deviating from the control sample's aberrant end cfDNA fragmentation profile.

3. The method of claim 2, further comprising:

providing a plurality of control samples obtained from the subject;

extracting cfDNA from the control samples to obtain control cfDNA fragments;

performing whole genome sequencing on the control cfDNA fragments extracted from the sample to generate sequencing reads for the control cfDNA fragments;

pooling the sequencing reads for the control cfDNA fragments;

determining from the pooled sequencing reads a distribution of start and end sites of the cfDNA fragments;

determining a nucleotide frequency at start sites and end sites of the cfDNA fragments thereby identifying aberrant fraction ends;

determining an aberrant ends fraction of the cfDNA fragments from the plurality of control samples to generate the aberrant end cfDNA fragmentation profile of the pooled control samples;

comparing the subject's cfDNA fragmentation profile to the pooled control samples' aberrant end cfDNA fragmentation profile; and

detecting the presence of disease or an abnormal cell type in the subject based on the subject's aberrant end cfDNA fragmentation profile deviating from the pooled control samples' aberrant end cfDNA fragmentation profile.

4. A method of detecting a cancer subtype in a subject using a cfDNA fragmentation profile of a subject, the method comprising the steps of:

providing a sample obtained from the subject;

extracting cfDNA from the sample to obtain cfDNA fragments;

performing whole genome sequencing on the cfDNA fragments extracted from the sample to generate sequencing reads for the cfDNA fragments;

determining from the sequencing reads a distribution of start and end sites of the cfDNA fragments, thereby identifying a fragmentation pattern of cfDNA fragments;

determining a nucleotide frequency at start sites and end sites of the cfDNA fragments relative to a nucleotide frequency at start sites and end sites of a control sample, thereby identifying aberrant fraction ends in the sample from the subject;

determining an aberrant ends fraction of the cfDNA fragments from the sample to generate the aberrant end cfDNA fragmentation profile of the subject;

comparing the subject's aberrant end cfDNA fragmentation profile to the aberrant end cfDNA fragmentation profile from the control sample;

creating a nucleosome map that identifies nucleosome peaks in the sample using the fragmentation pattern of cfDNA fragments of the subject;

comparing the nucleosome map of the subject to a nucleosome map that identifies the nucleosome peaks for the control sample, whereby deviation in peak position in the fragments from the subject sample and control sample can indicate the presence of a cancer subtype; and

detecting the presence of a cancer subtype in the subject based on differences between the aberrant end cfDNA fragmentation profile and the nucleosome map for the sample and the control sample.

5. The method of claim 4, further comprising:

providing a plurality of reference samples;

extracting cfDNA from the reference samples to obtain reference cfDNA fragments;

performing whole genome sequencing on the reference cfDNA fragments to generate sequencing reads for the reference cfDNA fragments;

pooling the sequencing reads for the reference cfDNA fragments;

determining from the pooled sequencing reads a distribution of start and end sites of the pooled cfDNA fragments;

determining a nucleotide frequency at start sites and end sites of the cfDNA fragments thereby identifying aberrant fraction ends; and

determining an aberrant ends fraction of the cfDNA fragments from the plurality of control samples to generate the aberrant end cfDNA fragmentation profile of the pooled control samples; and

determining the cancer subtype based on the fragmentation profile of the aberrant end cfDNA fragments deviating from the pooled reference fragmentation profile.

6. A method of detecting a subject's response to a treatment using a cfDNA fragmentation profile of a subject, the method comprising the steps of:

providing a first sample obtained from the subject prior to administering a treatment to the subject;

providing a second sample obtained from the subject after administering the treatment to the subject;

extracting cfDNA from the first sample to obtain cfDNA fragments;

extracting cfDNA from the second sample to obtain cfDNA fragments;

performing whole genome sequencing on the first and second sets of cfDNA fragments extracted from the samples to generate sequencing reads for the first and second set of cfDNA fragments;

determining from the sequencing reads a distribution of start and end sites of the cfDNA fragments;

determining a nucleotide frequency at start sites and end sites of the cfDNA fragments relative to a nucleotide frequency at start sites and end sites of a control sample, thereby identifying aberrant fraction ends in the samples from the subject;

determining an aberrant ends fraction of the cfDNA fragments from the sample to generate the subject's first and second aberrant end cfDNA fragmentation profile;

comparing the subject's first and second fragmentation profiles to a fragmentation profile of a reference sample; and

determining the subject's response to the treatment based on the similarity or difference in the subject's first and second cfDNA fragmentation profile compared to a reference sample cfDNA fragmentation profile.

7. The method of claim 6, further comprising determining the subject's response to the treatment based on the subject's second cfDNA fragmentation profile deviating from the subject's first cfDNA fragmentation profile.

8. The method of any one of claims 1 to 7, wherein detecting the fragmentation profile of the cfDNA fragments or detecting the first and second fragmentation profiles further comprises determining a median fragment length of the cfDNA fragments.

9. The method of claim 8, further comprising comparing the median fragment length of the cfDNA fragments to chromatin states of a selected cell type.

10. The method of any one of claims 1 to 9, wherein detecting the fragmentation profile of the cfDNA fragments or detecting the first and second fragmentation profiles further comprises determining coverage of cfDNA fragments at transcription start sites.

11. The method of claim 10, further comprising comparing the coverage of cfDNA fragments at transcription start sites to gene expression in a selected cell type or comparing coverage of the first and second sets of cfDNA fragments at transcription start sites with gene expression in a selected cell type.

12. The method of any one of claims 3 and 5-11, wherein the one or more control samples or the one or more reference samples are from healthy control subjects or from one or more patients having the same cancer subtype.

13. The method of any one of claims 2, 3, and 8-12, wherein the disease is cancer.

**Patentansprüche**

1. Verfahren zum Detektieren eines Fragmentierungsprofils zellfreier DNA (cfDNA) mit aberrierenden Enden in einem Subjekt, wobei das Verfahren die folgenden Schritte umfasst:

Bereitstellen einer Probe, die von dem Subjekt erlangt wurde;

Extrahieren von cfDNA aus der Probe, um cfDNA-Fragmente zu erlangen;

Durchführen einer Ganzgenomsequenzierung an den cfDNA-Fragmenten, die aus der Probe extrahiert wurden, um Sequenzauslesungen für die cfDNA-Fragmente zu erzeugen; und

Bestimmen, anhand der Sequenzauslesungen, einer Verteilung von Start- und Endstellen der cfDNA-Fragmente;

Bestimmen einer Nukleotidfrequenz an Startstellen und Endstellen der cfDNA-Fragmente relativ zu einer Nukleotidfrequenz an Startstellen und Endstellen einer Kontrollprobe, wodurch aberrierende Fraktionsenden in der Probe von dem Subjekt identifiziert werden; und

Bestimmen einer Fraktion aberrierender Enden der cfDNA-Fragmente aus der Probe, um das Fragmentierungsprofil von cfDNA mit aberrierenden Enden des Subjekts zu erzeugen.

2. Verfahren zum Detektieren einer Krankheit oder eines anormalen Zelltyps in einem Subjekt unter Verwendung eines Fragmentierungsprofils von cfDNA mit aberrierenden Enden eines Subjekts, wobei das Verfahren die folgenden Schritte umfasst:

Bereitstellen einer Probe, die von dem Subjekt erlangt wurde;

Extrahieren von cfDNA aus der Probe, um cfDNA-Fragmente zu erlangen;

Durchführen einer Ganzgenomsequenzierung an den cfDNA-Fragmenten, die aus der Probe extrahiert wurden, um Sequenzauslesungen für die cfDNA-Fragmente zu erzeugen;

Bestimmen, anhand der Sequenzauslesungen, einer Verteilung von Start- und Endstellen der cfDNA-Fragmente;

Bestimmen einer Nukleotidfrequenz an Startstellen und Endstellen der cfDNA-Fragmente relativ zu einer Nukleotidfrequenz an Startstellen und Endstellen einer Kontrollprobe, wodurch aberrierende Fraktionsenden in der Probe von dem Subjekt identifiziert werden;

Bestimmen einer Fraktion aberrierender Enden der cfDNA-Fragmente aus der Probe, um das Fragmentierungsprofil von cfDNA mit aberrierenden Enden des Subjekts zu erzeugen;

Vergleichen des Fragmentierungsprofils von cfDNA mit aberrierenden Enden des Subjekts mit einem Fragmentierungsprofil von cfDNA mit aberrierenden Enden aus einer Kontrollprobe; und

Detektieren des Vorhandenseins einer Krankheit oder eines anormalen Zelltyps in dem Subjekt basierend darauf, dass das Fragmentierungsprofil von cfDNA mit aberrierenden Enden des Subjekts von dem Fragmentierungsprofil von cfDNA mit aberrierenden Enden einer Kontrollprobe abweicht.

3. Verfahren nach Anspruch 2, das ferner Folgendes umfasst:

Bereitstellen einer Vielzahl von Kontrollproben, die von dem Subjekt erlangt wurden;

Extrahieren von cfDNA aus den Kontrollproben, um Kontroll-cfDNA-Fragmente zu erlangen;

Durchführen einer Ganzgenomsequenzierung an den Kontroll-cfDNA-Fragmenten, die aus der Probe extrahiert wurden, um Sequenzauslesungen für die Kontroll-cfDNA-Fragmente zu erzeugen;

Zusammenlegen der Sequenzauslesungen für die Kontroll-cfDNA-Fragmente;

Bestimmen, anhand der zusammengelegten Sequenzauslesungen, einer Verteilung von Start- und Endstellen der cfDNA-Fragmente;

Bestimmen einer Nukleotidfrequenz an Startstellen und Endstellen der cfDNA-Fragmente, wodurch aberrierende Fraktionsenden identifiziert werden;

Bestimmen einer Fraktion aberrierender Enden der cfDNA-Fragmente anhand der Vielzahl von Kontrollproben, um das Fragmentierungsprofil von cfDNA mit aberrierenden Enden der zusammengelegten Kontrollproben zu erzeugen;

Vergleichen des Fragmentierungsprofils von cfDNA des Subjekts mit dem Fragmentierungsprofil von cfDNA mit aberrierenden Enden aus den zusammengelegten Kontrollproben; und

Detektieren des Vorhandenseins einer Krankheit oder eines anormalen Zelltyps in dem Subjekt basierend darauf, dass das Fragmentierungsprofil von cfDNA mit aberrierenden Enden des Subjekts von dem Fragmentierungsprofil von cfDNA mit aberrierenden Enden der zusammengelegten Kontrollproben abweicht.

4. Verfahren zum Detektieren eines Krebssubtyps in einem Subjekt unter Verwendung eines cfDNA-Fragmentierungsprofils eines Subjekts, wobei das Verfahren die folgenden Schritte umfasst:

Bereitstellen einer Probe, die von dem Subjekt erlangt wurde;

Extrahieren von cfDNA aus der Probe, um cfDNA-Fragmente zu erlangen;

Durchführen einer Ganzgenomsequenzierung an den cfDNA-Fragmenten, die aus der Probe extrahiert wurden, um Sequenzauslesungen für die cfDNA-Fragmente zu erzeugen;

Bestimmen, anhand der Sequenzauslesungen, einer Verteilung von Start- und Endstellen der cfDNA-Fragmente, wodurch ein Fragmentierungsmuster der cfDNA-Fragmente identifiziert wird;

Bestimmen einer Nukleotidfrequenz an Startstellen und Endstellen der cfDNA-Fragmente relativ zu einer Nukleotidfrequenz an Startstellen und Endstellen einer Kontrollprobe, wodurch aberrierende Fraktionsenden in der Probe von dem Subjekt identifiziert werden;

Bestimmen einer Fraktion aberrierender Enden der cfDNA-Fragmente aus der Probe, um das Fragmentierungsprofil von cfDNA mit aberrierenden Enden des Subjekts zu erzeugen;

Vergleichen des Fragmentierungsprofils von cfDNA mit aberrierenden Enden des Subjekts mit dem Fragmentierungsprofil von cfDNA mit aberrierenden Enden aus der Kontrollprobe;

Erschaffen einer Nukleosom-Abbildung, die Nukleosom-Spitzen in der Probe unter Verwendung des Fragmentierungsmusters von cfDNA-Fragmenten des Subjekts identifiziert;

Vergleichen der Nukleosom-Abbildung des Subjekts zu einer Nukleosom-Abbildung, welche die Nukleosom-Spitzen für die Kontrollprobe identifiziert, wobei Abweichungen in Spitzenpositionen in den Fragmenten von der Subjektprobe und der Kontrollprobe das Vorhandensein eines Krebssubtyps anzeigen können; und

Detektieren des Vorhandenseins eines Krebssubtyps in dem Subjekt basierend auf Unterschieden zwischen dem Fragmentierungsprofil von cfDNA mit aberrierenden Enden und der Nukleosom-Abbildung für die Probe und die Kontrollprobe.

5. Verfahren nach Anspruch 4, das ferner Folgendes umfasst:

Bereitstellen einer Vielzahl von Referenzproben;

Extrahieren von cfDNA aus den Referenzproben, um ReferenzcfDNA-Fragmente zu erlangen;

Durchführen einer Ganzgenomsequenzierung an den ReferenzcfDNA-Fragmenten, um Sequenzauslesungen für die Referenz-cfDNA-Fragmente zu erzeugen;

Zusammenlegen der Sequenzauslesungen für die ReferenzcfDNA-Fragmente;

Bestimmen, anhand der zusammengelegten Sequenzauslesungen, einer Verteilung von Start- und Endstellen der zusammengelegten cfDNA-Fragmente;

Bestimmen einer Nukleotidfrequenz an Startstellen und Endstellen der cfDNA-Fragmente, wodurch aberrierende Fraktionsenden identifiziert werden; und

Bestimmen einer Fraktion aberrierender Enden der cfDNA-Fragmente anhand der Vielzahl von Kontrollproben, um das Fragmentierungsprofil von cfDNA mit aberrierenden Enden der zusammengelegten Kontrollproben zu erzeugen; und

Bestimmen des Krebssubtyps basierend darauf, dass das Fragmentierungsprofil der cfDNA-Fragmente mit aberrierenden Enden von dem zusammengelegten Referenz-Fragmentierungsprofil abweicht.

6. Verfahren zum Detektieren eines Ansprechens eines Subjekts auf eine Behandlung unter Verwendung eines cfDNA-Fragmentierungsprofils eines Subjekts, wobei das Verfahren die folgenden Schritte umfasst:

Bereitstellen einer ersten Probe, die von dem Subjekt vor Verabreichen einer Behandlung an das Subjekt erlangt wurde;

Bereitstellen einer zweiten Probe, die von dem Subjekt nach Verabreichen der Behandlung an das Subjekt erlangt wurde;

Extrahieren von cfDNA aus der ersten Probe, um cfDNA-Fragmente zu erlangen;

Extrahieren von cfDNA aus der zweiten Probe, um cfDNA-Fragmente zu erlangen;

Durchführen einer Ganzgenomsequenzierung an dem ersten und dem zweiten Satz von cfDNA-Fragmenten, die aus den Proben extrahiert wurden, um Sequenzauslesungen für den ersten und den zweiten Satz von cfDNA-Fragmenten zu erzeugen;

Bestimmen, anhand der Sequenzauslesungen, einer Verteilung von Start- und Endstellen der cfDNA-Fragmente;

Bestimmen einer Nukleotidfrequenz an Startstellen und Endstellen der cfDNA-Fragmente relativ zu einer Nukleotidfrequenz an Startstellen und Endstellen einer Kontrollprobe, wodurch aberrierende Fraktionsenden in den Proben von dem Subjekt identifiziert werden;

Bestimmen einer Fraktion aberrierender Enden der cfDNA-Fragmente aus der Probe, um das erste und das zweite Fragmentierungsprofil von cfDNA mit aberrierenden Enden des Subjekts zu erzeugen;

Vergleichen des ersten und des zweiten Fragmentierungsprofils des Subjekts mit einem Fragmentierungsprofil einer Referenzprobe; und

EP 3 997 242 B1

Bestimmen des Ansprechens des Subjekts auf die Behandlung basierend auf Ähnlichkeit oder Unterschied in dem ersten und dem zweiten cfDNA-Fragmentierungsprofil des Subjekts im Vergleich zu einem cfDNA-Fragmentierungsprofil einer Referenzprobe.

7. Verfahren nach Anspruch 6, ferner umfassend das Bestimmen des Ansprechens des Subjekts auf die Behandlung basierend darauf, dass das zweite cfDNA-Fragmentierungsprofil des Subjekts von dem ersten cfDNA-Fragmentierungsprofil des Subjekts abweicht.

8. Verfahren nach einem der Ansprüche 1 bis 7, wobei das Detektieren des Fragmentierungsprofils der cfDNA-Fragmente oder das Detektieren des ersten und des zweiten Fragmentierungsprofils ferner Bestimmen einer mittleren Fragmentlänge der cfDNA-Fragmente umfasst.

9. Verfahren nach Anspruch 8, ferner umfassend Vergleichen der mittleren Fragmentlänge der cfDNA-Fragmente mit Chromatinzuständen eines ausgewählten Zelltyps.

10. Verfahren nach einem der Ansprüche 1 bis 9, wobei das Detektieren des Fragmentierungsprofils der cfDNA-Fragmente oder das Detektieren des ersten und des zweiten Fragmentierungsprofils ferner Bestimmen einer Abdeckung von cfDNA-Fragmenten an Transkriptionsstartstellen umfasst.

11. Verfahren nach Anspruch 10, ferner umfassend Vergleichen der Abdeckung von cfDNA-Fragmenten an Transkriptionsstartstellen mit Genexpression in einem ausgewählten Zelltyp oder Vergleichen von Abdeckung des ersten und des zweiten Satzes von cfDNA-Fragmenten an Transkriptionsstartstellen mit Genexpression in einem ausgewählten Zelltyp.

12. Verfahren nach einem der Ansprüche 3 und 5-11, wobei die eine oder die mehreren Kontrollproben oder die eine oder die mehreren Referenzproben von gesunden Kontrollsubjekten oder von einem oder mehreren Patienten, die den gleichen Krebssubtyp aufweisen, stammen.

13. Verfahren nach einem der Ansprüche 2, 3 und 8-12, wobei es sich bei der Krankheit um Krebs handelt.

**Revendications**

1. Procédé de détection d'un profil de fragmentation de l'ADN acellulaire (cfADN) à extrémités aberrantes d'un sujet, le procédé comprenant les étapes consistant à :

fournir un échantillon obtenu à partir du sujet ;
extraire le cfADN à partir de l'échantillon pour obtenir des fragments de cfADN ;
réaliser un séquençage du génome entier sur les fragments de cfADN extraits à partir de l'échantillon pour générer des lectures de séquençage pour les fragments de cfADN ; et
déterminer à partir des lectures de séquençage une distribution de sites de début et de fin des fragments de cfADN ; déterminer une fréquence nucléotidique au niveau des sites de début et des sites de fin des fragments de cfADN par rapport à une fréquence nucléotidique au niveau des sites de début et des sites de fin d'un échantillon témoin, identifiant ainsi des extrémités de fraction aberrantes dans l'échantillon provenant du sujet ; et
déterminer une fraction d'extrémités aberrantes des fragments de cfADN à partir de l'échantillon pour générer le profil de fragmentation de cfADN à extrémités aberrantes du sujet.

2. Procédé de détection d'une maladie ou d'un type de cellule anormale chez un sujet en utilisant un profil de fragmentation de cfADN à extrémités aberrantes d'un sujet, le procédé comprenant les étapes consistant à :

fournir un échantillon obtenu à partir du sujet ;
extraire le cfADN à partir de l'échantillon pour obtenir des fragments de cfADN ;
réaliser un séquençage du génome entier sur les fragments de cfADN extraits à partir de l'échantillon pour générer des lectures de séquençage pour les fragments de cfADN ;
déterminer à partir des lectures de séquençage une distribution de sites de début et de fin des fragments de cfADN ;
déterminer une fréquence nucléotidique au niveau des sites de début et des sites de fin des fragments de cfADN par rapport à une fréquence nucléotidique au niveau des sites de début et des sites de fin d'un échantillon témoin,

24

identifiant ainsi des extrémités de fraction aberrantes dans l'échantillon provenant du sujet ;

déterminer une fraction d'extrémités aberrantes des fragments de cfADN à partir de l'échantillon pour générer le profil de fragmentation de cfADN à extrémités aberrantes du sujet ;

comparer le profil de fragmentation de cfADN à extrémités aberrantes du sujet à un profil de fragmentation de cfADN à extrémités aberrantes provenant d'un échantillon témoin ; et

détecter la présence d'une maladie ou d'un type de cellule anormale chez le sujet sur la base du profil de fragmentation de cfADN à extrémités aberrantes du sujet s'écartant du profil de fragmentation de cfADN à extrémités aberrantes de l'échantillon témoin.

3. Procédé selon la revendication 2, comprenant en outre :

la fourniture d'une pluralité d'échantillons témoins obtenus à partir du sujet ;

l'extraction du cfADN à partir des échantillons témoins pour obtenir des fragments de cfADN témoins ;

la réalisation d'un séquençage du génome entier sur les fragments de cfADN témoins extraits à partir de l'échantillon pour générer des lectures de séquençage pour les fragments de cfADN témoins ;

le regroupement des lectures de séquençage pour les fragments de cfADN témoins ;

la détermination à partir des lectures de séquençage regroupées d'une distribution de sites de début et de fin des fragments de cfADN ;

la détermination d'une fréquence nucléotidique au niveau des sites de début et des sites de fin des fragments de cfADN identifiant ainsi des extrémités de fraction aberrantes ;

la détermination d'une fraction d'extrémités aberrantes des fragments de cfADN à partir de la pluralité d'échantillons témoins pour générer le profil de fragmentation de cfADN à extrémités aberrantes des échantillons témoins regroupés ;

la comparaison du profil de fragmentation de cfADN du sujet au profil de fragmentation de cfADN à extrémités aberrantes des échantillons témoins regroupés ; et

la détection de la présence d'une maladie ou d'un type de cellule anormale chez le sujet sur la base du profil de fragmentation de cfADN à extrémités aberrantes du sujet s'écartant du profil de fragmentation de cfADN à extrémités aberrantes des échantillons témoins regroupés.

4. Procédé de détection d'un sous-type de cancer chez un sujet en utilisant un profil de fragmentation de cfADN d'un sujet, le procédé comprenant les étapes consistant à :

fournir un échantillon obtenu à partir du sujet ;

extraire le cfADN de l'échantillon pour obtenir des fragments de cfADN ;

réaliser un séquençage du génome entier sur les fragments de cfADN extraits à partir de l'échantillon pour générer des lectures de séquençage pour les fragments de cfADN ;

déterminer à partir des lectures de séquençage une distribution de sites de début et de fin des fragments de cfADN, identifiant ainsi un motif de fragmentation de fragments de cfADN ;

déterminer une fréquence nucléotidique au niveau des sites de début et des sites de fin des fragments de cfADN par rapport à une fréquence nucléotidique au niveau des sites de début et des sites de fin d'un échantillon témoin, identifiant ainsi des extrémités de fraction aberrantes dans l'échantillon provenant du sujet ;

déterminer une fraction d'extrémités aberrantes des fragments de cfADN à partir de l'échantillon pour générer le profil de fragmentation de cfADN à extrémités aberrantes du sujet ;

comparer le profil de fragmentation de cfADN à extrémités aberrantes du sujet au profil de fragmentation de cfADN à extrémités aberrantes provenant de l'échantillon témoin ;

créer une carte de nucléosomes qui identifie des pics de nucléosomes dans l'échantillon en utilisant le motif de fragmentation de fragments de cfADN du sujet ;

comparer la carte de nucléosomes du sujet par rapport à une carte de nucléosomes qui identifie les pics de nucléosomes pour l'échantillon témoin, moyennant quoi un écart de position de pic dans les fragments provenant de l'échantillon du sujet et de l'échantillon témoin peut indiquer la présence d'un sous-type de cancer ; et

détecter la présence d'un sous-type de cancer chez le sujet sur la base de différences entre le profil de fragmentation de cfADN à extrémités aberrantes et la carte de nucléosomes pour l'échantillon et l'échantillon témoin.

5. Procédé selon la revendication 4, comprenant en outre :

la fourniture d'une pluralité d'échantillons de référence ;

l'extraction du cfADN des échantillons de référence pour obtenir des fragments de cfADN de référence ;

la réalisation d'un séquençage du génome entier sur les fragments de cfADN de référence pour générer des lectures de séquençage pour les fragments de cfADN de référence ;

le regroupement des lectures de séquençage pour les fragments de cfADN de référence ;

la détermination à partir des lectures de séquençage regroupées d'une distribution de sites de début et de fin des fragments de cfADN regroupés ;

la détermination d'une fréquence nucléotidique au niveau des sites de début et des sites de fin des fragments de cfADN identifiant ainsi des extrémités de fraction aberrantes ; et

la détermination d'une fraction d'extrémités aberrantes des fragments de cfADN à partir de la pluralité d'échantillons témoins pour générer le profil de fragmentation de cfADN à extrémités aberrantes des échantillons témoins regroupés ; et

la détermination du sous-type de cancer sur la base du profil de fragmentation des fragments de cfADN à extrémités aberrantes s'écartant du profil de fragmentation de référence regroupé.

6. Procédé de détection de la réponse d'un sujet à un traitement en utilisant un profil de fragmentation de cfADN d'un sujet, le procédé comprenant les étapes consistant à :

fournir un premier échantillon obtenu à partir du sujet avant l'administration d'un traitement au sujet ;

fournir un second échantillon obtenu à partir du sujet après l'administration du traitement au sujet ;

extraire le cfADN du premier échantillon pour obtenir des fragments de cfADN ;

extraire le cfADN du second échantillon pour obtenir des fragments de cfADN ;

réaliser un séquençage du génome entier sur les premier et second ensembles de fragments de cfADN extraits à partir des échantillons pour générer des lectures de séquençage pour les premier et second ensembles de fragments de cfADN ;

déterminer à partir des lectures de séquençage une distribution de sites de début et de fin des fragments de cfADN ;

déterminer une fréquence nucléotidique au niveau des sites de début et des sites de fin des fragments de cfADN par rapport à une fréquence nucléotidique au niveau des sites de début et des sites de fin d'un échantillon témoin, identifiant ainsi des extrémités de fraction aberrantes dans les échantillons provenant du sujet ;

déterminer une fraction d'extrémités aberrantes des fragments de cfADN à partir de l'échantillon pour générer les premier et second profils de fragmentation de cfADN à extrémités aberrantes du sujet ;

comparer les premier et second profils de fragmentation du sujet à un profil de fragmentation d'un échantillon de référence ; et

déterminer la réponse du sujet au traitement sur la base de la similitude ou de la différence dans les premier et second profils de fragmentation de cfADN du sujet par rapport à un profil de fragmentation de cfADN d'échantillon de référence.

7. Procédé selon la revendication 6, comprenant en outre la détermination de la réponse du sujet au traitement sur la base du second profil de fragmentation de cfADN du sujet s'écartant du premier profil de fragmentation de cfADN du sujet.

8. Procédé selon l'une quelconque des revendications 1 à 7, dans lequel la détection du profil de fragmentation des fragments de cfADN ou la détection des premier et second profils de fragmentation comprend en outre la détermination d'une longueur de fragment médiane des fragments de cfADN.

9. Procédé selon la revendication 8, comprenant en outre la comparaison de la longueur de fragment médiane des fragments de cfADN à des états de chromatine d'un type de cellule sélectionné.

10. Procédé selon l'une quelconque des revendications 1 à 9, dans lequel la détection du profil de fragmentation des fragments de cfADN ou la détection des premier et second profils de fragmentation comprend en outre la détermination de la couverture de fragments de cfADN au niveau de sites de début de transcription.

11. Procédé selon la revendication 10, comprenant en outre la comparaison de la couverture de fragments de cfADN au niveau de sites de début de transcription à l'expression génique dans un type de cellule sélectionné ou la comparaison de la couverture des premier et second ensembles de fragments de cfADN au niveau de sites de début de transcription avec l'expression génique dans un type de cellule sélectionné.

12. Procédé selon l'une quelconque des revendications 3 et 5 à 11, dans lequel les un ou plusieurs échantillons témoins ou les un ou plusieurs échantillons de référence proviennent de sujets témoins sains ou d'un ou plusieurs patients ayant le même sous-type de cancer.

13. Procédé selon l'une quelconque des revendications 2, 3, et 8 à 12, dans lequel la maladie est un cancer.

FIG. 1A

FIG. 1B

FIG. 1C

FIG. 2A

FIG. 2B

FIG. 2C

FIG. 2D

FIG. 2E

FIG. 2F

FIG. 2G

FIG. 2H

FIG. 2I

FIG. 3A

FIG. 3B

FIG. 3C

FIG. 3D

FIG. 3E

FIG. 3F

FIG. 3G

EXPRESSION BINS ----FPKM≤0.1 ——FPKM>0.1 AND ≤1 ···FPKM>1 AND ≤8 ——FPKM>8

FIG. 4A

FIG. 4B

CELL TYPE ■ BONE MARROW, LYMPHOID, ☒ KIDNEY OR URINARY ☐ OTHER
OR MYELOID CELLS     BLADDER CELLS

FIG. 4C

FIG. 4D

FIG. 4E

FIG. 4F

FRAGMENT ORIENTATION ——— START POS ——— END POS

FIG. 5A

EP 3 997 242 B1

FIG. 5B

FIG. 5C

FIG. 6A

FIG. 6B

FIG. 6C

FIG. 6D

COPY NUMBER ABERRATIONS IN TUMOR BIOPSY

FIG. 7A

FIG. 7B

COPY NUMBER ABERATIONS IN CORRESPONDING URINE CFDNA SAMPLE

TUMOR FRACTION: 0, PLOIDY: 2

FIG. 7C

EP 3 997 242 B1

FIG. 8A

FIG. 8B

FIG. 8C

FIG. 8D

FRAGMENT SIZE

FIG. 8E

FIG. 9

FIG. 10

EP 3 997 242 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62872234
- US 62888997

**Non-patent literature cited in the description**

- **RAYMOND et al.** *PLOS ONE*, 2017
- **MURTAZA, M.** ; **CALDAS, C.** Nucleosome mapping in plasma DNA predicts cancer gene expression. *Nat Genet*, 2016, vol. 48, 1105-1106
- **CRISTIANO, S. et al.** Genome-wide cell-free DNA fragmentation in patients with cancer. *Nature*, 2019
- **SNYDER, M. W** ; **KIRCHER, M.** ; **HILL, A. J.** ; **DAZA, R. M.** ; **SHENDURE, J**. Cell-free DNA Comprises an In Vivo Nucleosome Footprint that Informs Its Tissues-Of-Origin. *Cell*, 2016, vol. 164, 57-68
- **ULZ, P. et al.** Inferring expressed genes by whole-genome sequencing of plasma DNA. *Nat Genet*, 2016, vol. 48, 1273-1278
- **MOULIERE, F. et al.** Enhanced detection of circulating tumor DNA by fragment size analysis.. *Sci Transl Med*, 2018, vol. 10
- **CHENG, T. H. T. et al.** Genomewide bisulfite sequencing reveals the origin and time-dependent fragmentation of urinary cfDNA. *Clin Biochem*, 2017, vol. 50, 496-501
- **TSUI, N. B. et al.** High resolution size analysis of fetal DNA in the urine of pregnant women by paired-end massively parallel sequencing. *PLoS One*, 2012, vol. 7, e48319
- **LO, Y. M. et al.** Maternal plasma DNA sequencing reveals the genome-wide genetic and mutational profile of the fetus. *Sci Transl Med*, 2010, vol. 2, 61-91
- **THASTROM, A** ; **BINGHAM, L. M.** ; **WIDOM, J.** Nucleosomal locations of dominant DNA sequence motifs for histone-DNA interactions and nucleosome positioning. *J Mol Biol*, 2004, vol. 338, 695-709
- **GAFFNEY, D. J. et al.** Controls of nucleosome positioning in the human genome.. *PLoS Genet*, 2012, vol. 8, e1003036
- **CHANDRANANDA, D** ; **THORNE, N. P.** ; **BAHLO, M**. High-resolution characterization of sequence signatures due to non-random cleavage of cell-free DNA.. *BMC Med Genomics*, 2015, vol. 8, 29
- **CHEN, S. et al.** A Study of Cell-free DNA Fragmentation Pattern and Its Application in DNA Sample Type Classification. *IEEE/ACM Trans Comput Biol Bioinform*, 2017
- **SERPAS, L. et al.** Dnase1l3 deletion causes aberrations in length and end-motif frequencies in plasma DNA.. *Proc Natl Acad Sci U S A*, 2019, vol. 116, 641-649
- **NADANO, D** ; **YASUDA, T.** ; **KISHI, K**. Measurement of deoxyribonuclease I activity in human tissues and body fluids by a single radial enzyme-diffusion method.. *Clin Chem*, 1993, vol. 39, 448-452
- **NAPIREI, M** ; **LUDWIG, S** ; **MEZRHAB, J** ; **KLOCKL, T.** ; **MANNHERZ, H. G**. Murine serum nucleases--contrasting effects of plasmin and heparin on the activities of DNase1 and DNase1-like 3 (DNase1l3). *FEBS J*, 2009, vol. 276, 1059-1073
- **BALDI, S** ; **KREBS, S** ; **BLUM, H.** ; **BECKER, P. B**. Genome-wide measurement of local nucleosome array regularity and spacing by nanopore sequencing.. *Nat Struct Mol Biol*, 2018, vol. 25, 894-901
- **MAURANO, M. T. et al.** Systematic localization of common disease-associated variation in regulatory DNA.. *Science*, 2012, vol. 337, 1190-1195
- **SUN, K. et al.** Orientation-aware plasma cell-free DNA fragmentation analysis in open chromatin regions informs tissue of origin.. *Genome Res*, 2019, vol. 29, 418-427
- **LIU, Y. et al.** Spatial co-fragmentation pattern of cell-free DNA recapitulates in vivo chromatin organization and identifies tissues-of-origin. *bioRxiv*, 2019, 564773
- **LU, T.** ; **LI, J**. Clinical applications of urinary cell-free DNA in cancer: current insights and promising future.. *Am J Cancer Res*, 2017, vol. 7, 2318-2332
- **FUJII, T. et al.** Mutation-Enrichment Next-Generation Sequencing for Quantitative Detection of KRAS Mutations in Urine Cell-Free DNA from Patients with Advanced Cancers.. *Clin Cancer Res*, 2017, vol. 23, 3657-3666
- **MARKUS, H. et al.** Evaluation of pre-analytical factors affecting plasma DNA analysis.. *Sci Rep*, 2018, vol. 8, 7375
- **LI, H**. Aligning sequence reads, clone sequences and assembly contigs with BWA-MEM. *arXiv preprint arXiv:1303.3997*, 2013

- **LI, H. et al.** The Sequence Alignment/Map format and SAMtools.. *Bioinformatics*, 2009, vol. 25, 2078-2079
- **QUINLAN, A. R.** ; **HALL, I. M.** BEDTools: a flexible suite of utilities for comparing genomic features.. *Bioinformatics*, 2010, vol. 26, 841-842
- **FAVERO, F. et al.** Sequenza: allele-specific copy number and mutation profiles from tumor sequencing data.. *Ann Oncol*, 2015, vol. 26, 64-70
- **BATTH, T. S.** ; **FRANCAVILLA, C** ; **OLSEN, J. V**. Off-line high-pH reversed-phase fractionation for in-depth phosphoproteomics.. *J Proteome Res*, 2014, vol. 13, 6176-6186
- **THE, M** ; **MACCOSS, M. J.** ; **NOBLE, W. S** ; **KALL, L**. Fast and Accurate Protein False Discovery Rates on Large-Scale Proteomics Data Sets with Percolator 3.0. *J Am Soc Mass Spectrom*, 2016, vol. 27, 1719-1727